# EUROPEAN PATENT SPECIFICATION

(11) **EP 1 805 680 B1**
(45) Date of publication and mention of the grant of the patent: **25.03.2015**
(21) Application number: 05807313.1
(22) Date of filing: 06.10.2005
(51) Int. Cl.: G06F 19/00, G01N 33/53, G01N 33/50

(54) **CHANNEL CURRENT CHEMINFORMATICS AND BIOENGINEERING METHODS FOR IMMUNOLOGICAL SCREENING, SINGLE-MOLECULE ANALYSIS, AND SINGLE-MOLECULAR-INTERACTION ANALYSIS**
KANALSTROM-CHEMINFORMATIK UND BIOTECHNISCHE VERFAHREN FÜR IMMUNOLOGISCHES SCREENING, EINZELMOLEKÜLANALYSE UND EINZELMOLEKULARINTERAKTIONSANALYSE
PROCEDES DE GENIE BIOLOGIQUE ET DE CHIMIO-INFORMATIQUE DU COURANT DE CANAL POUR CRIBLAGE IMMUNOLOGIQUE, ANALYSE DE MOLECULES INDIVIDUELLES, ET ANALYSES DES INTERACTIONS DE MOLECULES INDIVIDUELLES

(30) Priority: 06.10.2004 US 616274 P; 06.10.2004 US 616275 P; 06.10.2004 US 616276 P; 06.10.2004 US 616277 P
(43) Date of publication of application: 11.07.2007
(73) Proprietor: Board of Supervisors of Louisiana, New Orleans, LA 70112 (US); Board of Supervisors of Louisiana, New Orleans, LA 70122 (US)
(72) Inventor: WINTERS-HILT, Stephen, N., Mandeville, LA 70471 (US); PINCUS, Seth, H., New Orleans, LA 70118 (US)
(74) Representative: Andrae | Westendorp Patentanwälte Partnerschaft
(86) International application number: PCT/US2005/035933
(87) International publication number: WO 2006/041983

(56) References cited:
- WO-A-99/48927
- WO-A-02/079417
- WO-A-03/000899
- WO-A2-01/59453
- US-A1- 2003 099 951
- US-A1- 2003 104 428
- US-B1- 6 180 356
- US-B1- 6 627 067
- WINTERS-HILT STEPHEN ET AL: "Highly accurate classification of Watson-Crick basepairs on termini of single DNA molecules." BIOPHYSICAL JOURNAL. FEB 2003, vol. 84, no. 2 Pt 1, February 2003 (2003-02), pages 967-976, XP002383574 ISSN: 0006-3495 cited in the application
- ZHOU B Y ET AL: "Specific antibodies to the external vestibule of voltage-gated potassium channels block current." THE JOURNAL OF GENERAL PHYSIOLOGY. APR 1998, vol. 111, no. 4, April 1998 (1998-04), pages 555-563, XP002109760 ISSN: 0022-1295
- A.GEORGE, J. GALLOP, M.RASHID: "Kinetics of biomolecular interactions." EXPERT OPINION ON THERAPEUTIC PATENTS, vol. 7, no. 9, 1997, pages 947-963, XP002383575
- KAGAN B L ET AL: "Diphtheria toxin fragment forms large pores in phospholipid bilayer membranes." PROCEEDINGS OF THE NATIONAL ACADEMY OF SCIENCES OF THE UNITED STATES OF AMERICA. AUG 1981, vol. 78, no. 8, August 1981 (1981-08), pages 4950-4954, XP002394679 ISSN: 0027-8424
- GU LI-QUN ET AL: "Stochastic sensing of organic analytes by a pore-forming protein containing a molecular adapter", NATURE, vol. 398, no. 6729, 22 April 1999 (1999-04-22), pages 686-690, XP002252375,
- VERCOUTERE W ET AL: "Rapid discrimination among individual DNA hairpin molecules at single-nucleotide resolution using an ion channel", NATURE BIOTECHNOLOGY, NATURE PUBLISHING GROUP, NEW YORK, NY, US, vol. 19, no. 3, 1 March 2001 (2001-03-01), pages 248-252, XP002226143, ISSN: 1087-0156, DOI: 10.1038/85696
- W. A. VERCOUTERE: 'Discrimination among individual Watson-Crick base pairs at the termini of single DNA hairpin molecules' NUCLEIC ACIDS RESEARCH vol. 31, no. 4, 15 February 2003, pages 1311 - 1318, XP055026205 DOI: 10.1093/nar/gkg218 ISSN: 0305-1048
- STEPHEN WINTERS-HILT: 'Single-Molecule Biochemical Analysis Using Channel Current Cheminformatics' AIP CONFERENCE PROCEEDINGS vol. 800, 10 June 2005, pages 337 - 342, XP055026269 DOI: 10.1063/1.2138634 ISSN: 0094-243X
- Stephen Winters-Hilt: "Machine learning methods for channel current cheminformatics, biophysical analysis, and bioinformatics", 10 July 2003 (2003-07-10)

## Description

### TECHNICAL FIELD

The present invention pertains to the field of molecular chemistry, and in particular, to methods of molecular characterization and analysis using electrophoretically driven ion flow through one or more channels in a suitable substrate.

### REFERENCE TO RELATED APPLICATIONS

Claim is hereby made to the benefits of prior, co-pending United States Provisional Patent Applications 60/616,274, 60/616,275, 60/616,276 and 60/616,277, all filed an October 6,2004.

### SEQUENCE LISTING

A Sequence Listing, which lists the sequences identified by Sequence ID Number, corresponding to the Sequence ID Number used herein, accompanies this disclosure and is incorporated herein by reference.

When used herein, unless the context demands otherwise, the term "channel" is synonymous with the term "pore." A "conductive medium" means a medium capable of conducting an ionic flow.

### BACKGROUND

The notion of using channels as detection devices dates back to the Coulter counter, where pulses in channel flow were measured in order to count bacterial cells. Cell transport through the Coulter counter is driven by hydrostatic pressure - and interactions between the cells and the walls of the channel are ignored. Since its original formulation, channel sizes have reduced from millimeter scale to nanometer scale, and the detection mechanism has shifted from measurements of hydrostatically driven fluid flow to measurements of electrophoretically driven ion flow. Analytes observed via channel measurements are likewise reduced in scale, and are now at the scale of single biomolecules such as DNA and polypeptides. To a limited extent, some intramolecular, Angstrom-level, features are beginning to be resolved as well.

For nanoscopic channels, interactions between channel wall and translocating biomolecules can't, usually, be ignored. On the one hand this complicates analysis of channel blockade signals, on the other hand, tell-tale on-off kinetics are revealed for binding between analyte and channel, and this is what has allowed the probing of intramolecular structure on single DNA molecules.

US 2003/0099951 describes methods and devices for characterizing a duplex nucleic acid, e.g., a duplex DNA molecule. In the subject methods, a fluid conducting medium that includes a duplex nucleic acid molecule is contacted with a nanopore under influence of an applies electric field and the resulting changes in current through the nanopore caused by the duplex nucleic acid molecule are monitored.

GU Li-Qun et al. discloses stochastic sensing of organic analytes, wherein the stochastic sensing of organic molecules can be procured from α-haemolysin by equipping the channels with an internal, non-covalently bound molecular "adapter" which mediates channel blocking by the analyte (NATURE, vol. 398, no. 6729, 22 April 1999, pages 686-690).

Biophysicists and medical researchers have performed measurements of ion flow through single nanopores since the 1970's. It was they who first designed the sensitive patch clamp amplifiers needed for the picoampere ionic current measurements. Typically, these single channel techniques have been applied to low conductance, ion selective channels, such as gated potassium channels, but they have also been applied to larger channels involved in metabolite and macromolecule transport. The use of large biological pores as polymer sensors is a relatively new possibility that dates from the pioneering experiments of Bezrukov et al., circa 1994, and Kasianowicz et al., circa 1996. Bezrukov, S.M., I. Vodyanoy, V.A. Parsegian. 1994, Counting polymers moving through a single ion channel, Nature 370 (6457), pgs 279-281; Kasianowicz, J.J., E. Brandin, D. Branton, and D.W. Deamer, 1996, Characterization of Individual Polynucleotide Molecules Using a Membrane Channel, Proc. Natl. Acad. Sci. USA 93(24), 13770-73. This work proved that resistive pulse measurements, familiar from cell counting with the Coulter counter, could be reduced to the molecular scale and applied to polymers in solution. A seminal paper by Kasianowicz et al., circa 1996, then showed that individual DNA and RNA polymers could be detected via their translocation blockade of a nanoscale pore formed by a-hemolysin toxin. In such prior nanopore detection work, the data analysis problems were of a familiar "Coulter event" form - where the event was associated with a current blockade at a certain, fixed level. For this type of blockade signal, information on transitions between sub-blockades is often negligible.

Until now, there has been very little, if any, recognition of the wealth of information which might be obtained from current channel blockades with frequent transitions between distinct sub-blockades. The present invention involves not only the recognition of this information, but also techniques for obtaining the information and applying it in very useful ways.

### SUMMARY OF THE INVENTION

The present invention brings forth a novel modification of a channel current-based detection system, using what are herein termed auxiliary molecules and artificial intelligence (e.g., pattern recognition) software systems for the analysis of the data obtained. The auxiliary molecule employed produces a "toggling" blockade between several different levels (with two usually dominating). The resulting blockade signal for the auxiliary molecule by itself is no longer at approximately a fixed blockade level, but now consists of a telegraph-like blockade signal with stationary statistics. Upon binding of analyte to the auxiliary molecule the toggling signal is greatly altered, to one with different transition timing and different blockade residence levels. Building on this as a biosensing foundation requires sophisticated computational tools, such as Hidden Markov Models and Support Vector Machines (SVMs), but offers at least a hundred-fold improvement to the sensitivity of the device. This is because the events of analyte bound vs non-bound (detected or not) can now be discerned with the much greater information in the multiple-level residencies and transition timings. The sparse information provided by previous systems, with non-bound having one blockade level and bound having another, single, blockade level, presents a minimal amount of information when to compared to the present invention. Given the noise in the prior systems and the limited dynamic range for blockades of the open channel current, the systems devoid of an auxiliary molecule component are greatly restricted because they are not endowed with this sensitive timing information.

Thus, the present invention provides in one embodiment a method as described in claim 1.
In another embodiment of the invention, this method further comprises comparing the blockade channel current signal pattern, or the change in the blockade channel current signal pattern, to at least one blockade channel current signal pattern, or at least one change in the blockade channel current signal pattern, associated with at least one known condition, to thereby correlate the test condition with the known condition.

Also described herewith is a method for detecting antigens using at least one nanometer-scale channel through which an ionic current may flow, which channel is coupled to an antibody to form a blockade sufficient to modulate ionic current passing through the channel so as to provide a signal indicative of the blockade in the channel, thereby providing a signal which, when processed in accordance with the present invention, enables bio-sensing. Another aspect involves utilization of pattern recognition software specifically designed to analyze channel currents.

Furthermore, the method comprises drawing part of a single antibody into a single nanometer-scale channel, where only a single channel is established to conduct current through a membrane (e.g., an organic or solid-state membrane). A single-antibody blockade event forms the background channel current signal that is monitored for antigen binding events. An antigen-binding event results in a perturbation of the channel current signal of the antibody channel blockade, and is the basis of the detection observation.

The methods of this disclosure also can be applicable in a variety of ways where many channels are present, where each channel offers parallel conductance paths for the ionic current, and where each channel is exposed to antibody to establish a background collection of channel/antibody signals that is modifiable in the presence of antigen. Multiple antibody species can be present in this multi-channel aspect. Anything that can evoke an antibody response can be taken as the antigen or collection of antigens for which the bio-sensing is designed.

One application of the present disclosure is in the field of bio-defense. Thus, in accordance with one aspect, a fractionated bio-terror organism, such as, e.g., fractionated anthrax spores (i.e., pieces of protein, etc.), may be injected into a mouse to evoke a broad-spectrum antibody response. A collection of nanopore/antibody channel currents could then be prepared with those antibodies for detection of fractionated anthrax spores obtained via an air-concentrator with the same protein fractioning processes. Introduction of the fractionated air-concentrate when spores are present to the nanopore/antibody detector would then evoke a discernible indication of the presence of that microorganism.

Further described herewith is a method for identifying pore inhibiting agents for treatment against pore-forming toxins associated with disease, bacterial and viral infection, and chemical exposure. The method makes use of pattern recognition software specialized to analysis of channel currents. In each aspect a pore-forming toxin, or membrane-permeabilizing agent, is introduced into a membrane (typically a synthetic biological membrane, such as a lipid bilayer, although hybrid organic/solid state membranes may also be used). In addition, the lifetime of a single toxin channel is characterized prior to its disruption by the pore inhibiting agent being screened. The method also enables one to screen for agents that might prevent pore formation, by examination of the average time before channel formation under identical circumstances (pore forming toxin w/wo pore inhibiting agents).

In addition, multiple toxin channels may be introduced and disruptions to their cumulative currents are studied, or the toxin is introduced and the timescale of multi-channel formation is examined in the presence of pore inhibiting agent. The multi-pore aspects are applicable on very fast time-scales, while the single-pore aspects provide single-molecule information about the action of the pore inhibiting agents. The single-channel aspects can also be used to inform the design and statistical modeling of the multi-channel aspects in the screening process. Pore-inhibiting agents that can be studied via these aspects include antimicrobial peptides, the larger family of amphipathic molecules, and antibodies.

Medicines and vaccines that provide resistance to pore-forming and membrane permeabilizing toxins can be rapidly assayed using methods of this disclosure. Pore-forming toxins are often virulence factors associated with bacterial and viral infections, or with toxic chemical exposure. The medicines obtained can either provide resistance to pore-forming toxin prior to their pore-formation or act as treatment after exposure to pore-forming toxins.

Still described herewith is a method for identifying effective cytosolic antigen delivery mechanisms for use in evoking cytotoxic T lymphocyte (CTL) responses in organisms challenged by cytosolic virulence factors. In this particular method, pore-forming toxins are used that allow introduction of peptides and other molecules into the cytosol of their host cell. The method entails establishing a single such pore-forming toxin in a membrane followed by measurement of peptide (antigen) transmembrane transport via channel current measurements. This method make use of established procedures for attaching target antigen to the recognition sequence of virulence factors associated with the pore-forming toxin in the natural setting. Via this transmembrane transport mechanism, a number of antigen/recognition molecules can be assayed for effective use with the chosen pore-forming toxin, and thereby provide an aspect of a cytosolic antigen delivery assayer.

Medicines and vaccines that provide resistance to cytosolic virulence factors can be rapidly assayed with this approach. Pore-forming toxins and viruses (eg, HIV) are often associated with cytosolic virulence factors. Medicines and vaccines can provide treatment or resistance to such cytosolic virulence factors if an effective mechanism is devised for delivery of virulence-associated antigen to the cytosol of the host cell. Effective delivery of antigen to the cytosol is the first step in obtaining an effective agent for evoking a CTL response against virulence factors of the invading microorganism.

Yet another aspect of the present disclosure is a method for measuring the binding kinetics of an antibody to an associated antigen, and more generally for measuring the affinity of an antibody for a given antigen. The disclosure employs a nanometer-scale channel that is coupled to an antibody as an antibody characterization and antibody-antigen efficacy-screening tool. In one aspect, the method employs pattern recognition software specialized to analysis of channel currents. In another aspect, the method comprises drawing part of a single antibody into a single nanometer-scale channel. When only a single channel is conducting current through a membrane (e.g., a lipid bilayer or solid state membrane) the antibody blockade is a clearly discernible event, and forms the background channel current signal that is monitored (e.g., using a software-based signal processor) for antigen binding events. An antigen binding event then perturbs the channel current signal resulting from the antibody channel-blocking alone, and is the basis of the detection observation. The methods are also applicable in a variety of aspects when many channels are present (offering parallel conductance paths for the ionic current), where each channel is exposed to the same antibody to establish a background collection of channel/antibody signals that is modifiable in the presence of antigen that binds to that antibody. In either the single nanopore/antibody or multiple nanopore/antibody aspects this provides a platform for antibody characterization. With addition of antigen that led to the antibody obtained, the binding kinetics can be observed, and the strength (efficacy) of the antibody for that antigen assessed.

Through practice of the present invention, single antibody studies can be performed in a new way via the nanopore device. Of particular importance are studies of the antibody-antigen binding affinities for assessment of the antibody efficacy in medical treatments.

These and other embodiments, features and advantages of the present invention shall now become apparent from the ensuing detailed description of embodiments of the invention, the appended figures and accompanying claims.

### SUMMARY OF THE FIGURES

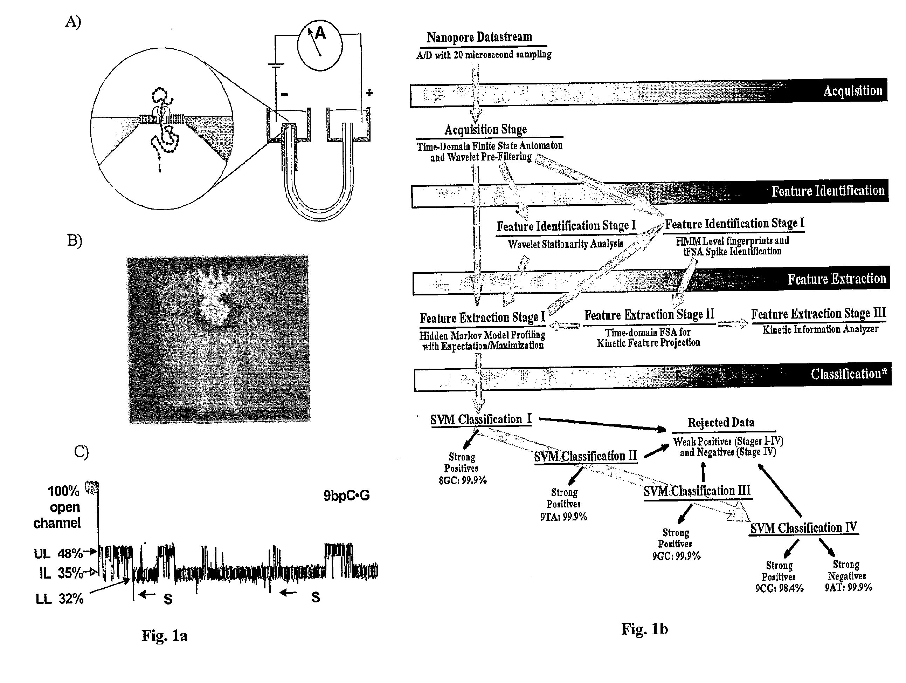

**Figure 1a****. (prior art)** (A) shows a nanopore device based on the α-hemolysin channel. It has been used for analysis of single DNA molecules, such as ssDNA, shown, and dsDNA, a nine base-pair DNA hairpin is shown in (B) superimposed on the channel geometry. The channel current blockade trace for the nine base-pair DNA hairpin blockade from (B) is shown in (C).
**Figure 1b** is a flow diagram illustrating the signal processing architecture that was used to classify DNA hairpins: Signal acquisition was performed using a time-domain, thresholding, Finite State Automaton, followed by adaptive pre-filtering using a wavelet-domain Finite State Automaton. Hidden Markov Model processing with Expectation-Maximization was used for feature extraction on acquired channel blockades. Classification was then done by Support Vector Machine on five DNA molecules: four DNA hairpin molecules with nine base-pair stem lengths that only differed in their blunt-ended DNA termini, and an eight base-pair DNA hairpin. The accuracy shown is obtained upon completing the 15^{th} single molecule sampling/classification (in approx. 6 seconds), where SVM-based rejection on noisy signals was employed.

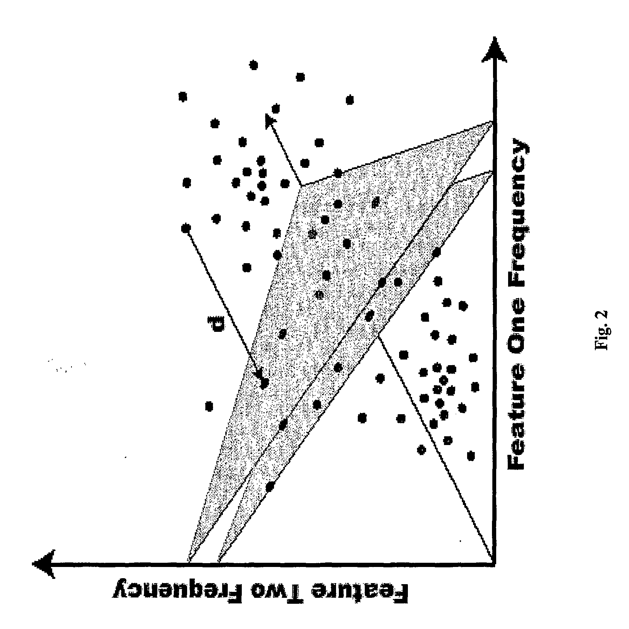

**Figure 2****.** A sketch of the hyperplane separability heuristic for SVM binary classification. An SVM is trained to find an optimal hyperplane that separates positive and negative instances, while also constrained by structural risk minimization (SRM) criteria, which here manifests as the hyperplane having a thickness, or "margin," that is made as large as possible in seeking a separating hyperplane. A benefit of using SRM is much less complication due to overfitting (a problem with Neural Network discrimination approaches).

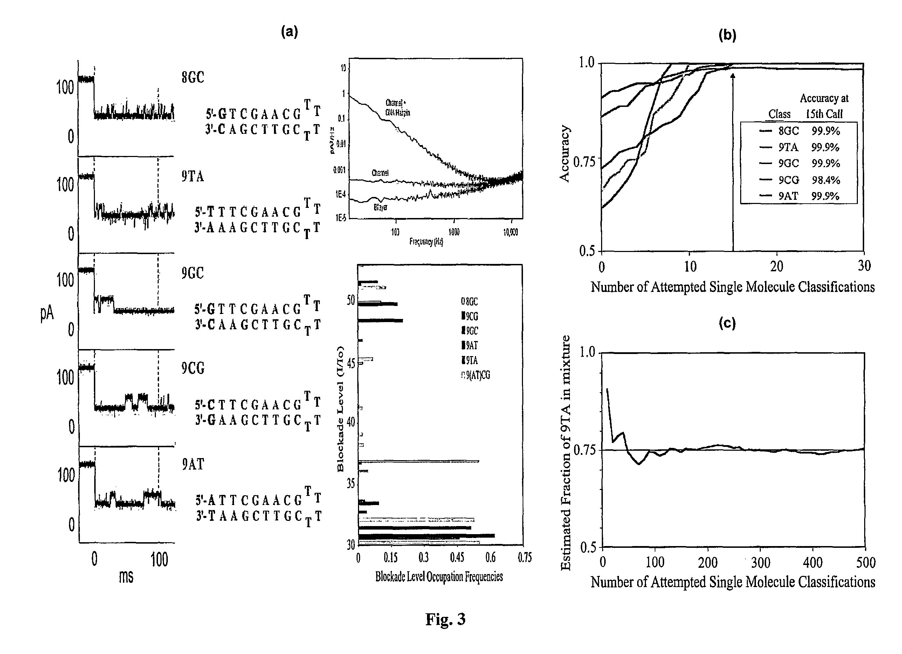

**Figure 3****.** (a) The channel current blockade signals observed when selected DNA hairpins are disposed within the channel. The left panel shows the five DNA hairpins, with sample blockades, that were used to test the sensitivity of the nanopore device. The top right panel shows the power spectral density for signals obtained. The bottom right panel shows the dominant blockades, and their frequencies, for the different hairpin molecules. (b) A graph showing the single-species classification prediction accuracy as the number of signal classification attempts increases (allowing increase in the rejection threshold). (c) A graph showing the prediction accuracy on 3:1 mixture of 9TA to 9GC DNA hairpins. 8GC has SEQ ID NO: 1. 9TA has SEQ ID NO: 2. 9GC has SEQ ID NO 3. 9CG has SEQ ID NO: 4. 9AT has SEQ ID NO 5.

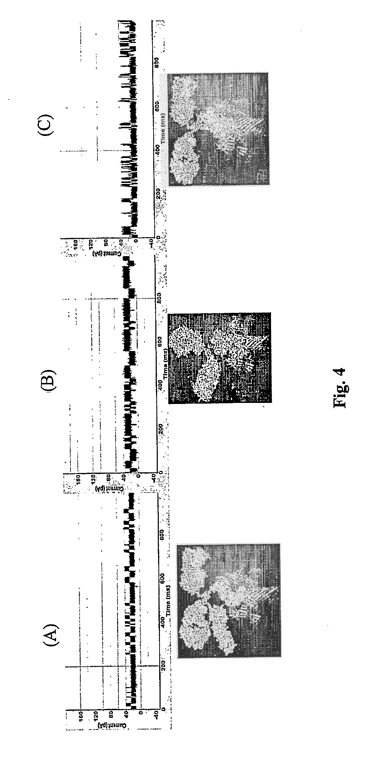

**Figure 4****.** Signal analysis from an experiment carried out in accordance with an embodiment of this invention, involving a single antibody but different blockade signals. (A) The lower-level (LL) blockade of this signal is not as deep as the others, and may correspond to antibody capture of a free heavy chain. (B) The LL blockade of this signal is about mid-way between those shown, so may correspond to capture a Fab arm. (C) The LL blockade of this signal is the deepest, and difficult to eject with voltage reversal (requiring several attempts), so may correspond to an entire Fc arm capture. Each graph shows the level of current in picoamps over time in milliseconds.

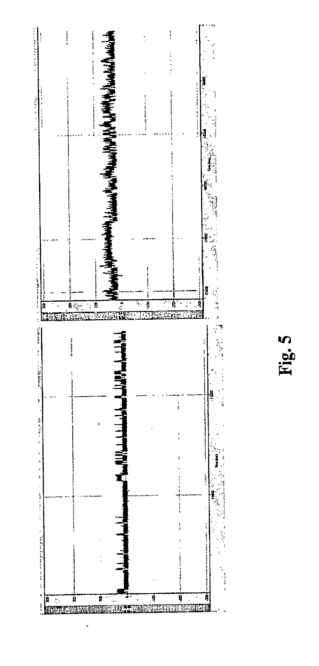

**Figure 5****.** Signal analysis from an experiment carried out in accordance with an embodiment of this invention, showing (A) Blockade due to T6 antibody and (B) T6 antibody blockade one minute after exposure to antigen. Each graph shows the level of current in picoamps over time in milliseconds.

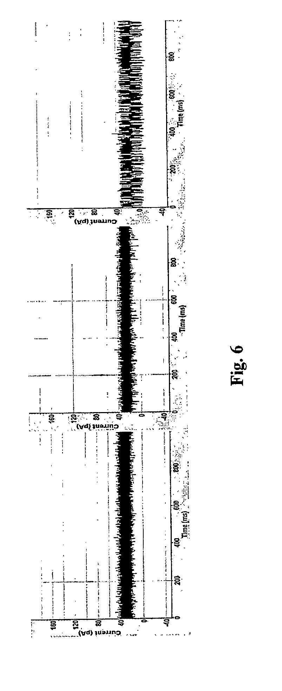

**Figure 6****.** (A) Channel current blockade signal where the blockade is produced by 9GC DNA hairpin with 20bp stem. (B) Channel current blockade signal where the blockade is produced by 9GC 20bp stem with magnetic bead attached. (C) Channel current blockade signal where the blockade is produced by c9GC 20bp stem with magnetic bead attached and driven by a laser beam chopped at 4Hz. Each graph shows the level of current in picoamps over time in milliseconds.

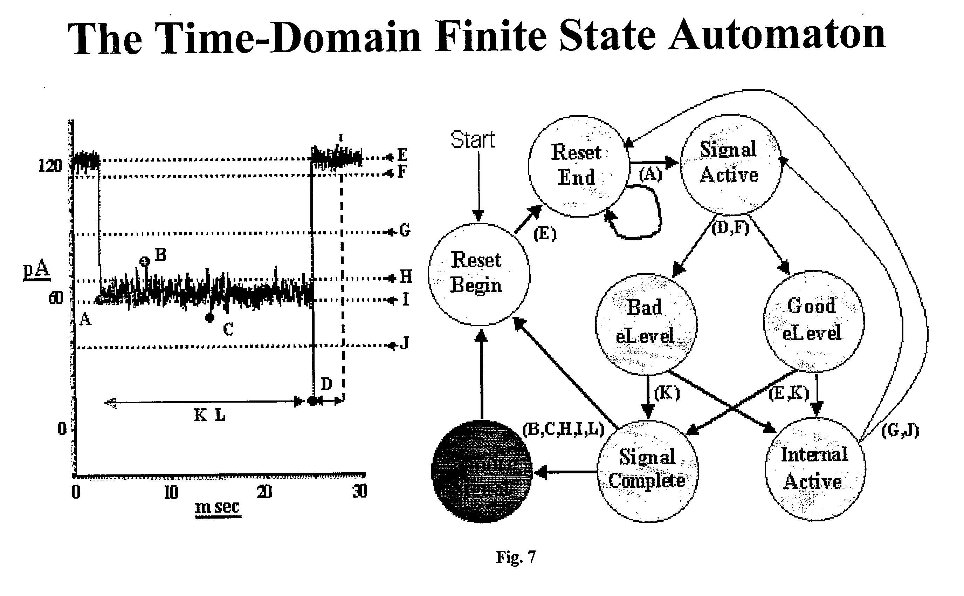

**Figure 7****.** Shows the architecture of the FSA employed in an embodiment of this invention (specific details on tuning parameters can be found in Winters-Hilt, S., W. Vercoutere, V.S. DeGuzman, D.W. Deamer, M. Akeson, and D. Haussler. 2003. Highly Accurate Classification of Watson-Crick Basepairs on Termini of Single DNA Molecules. Biophys. J. 84:967-976 (hereinafter referred to as "Winter-Hilt, et al. 2003"). Tuning on FSA parameters was done using a variety of heuristics, including tuning on statistical phase transitions and feature duration cutoffs.

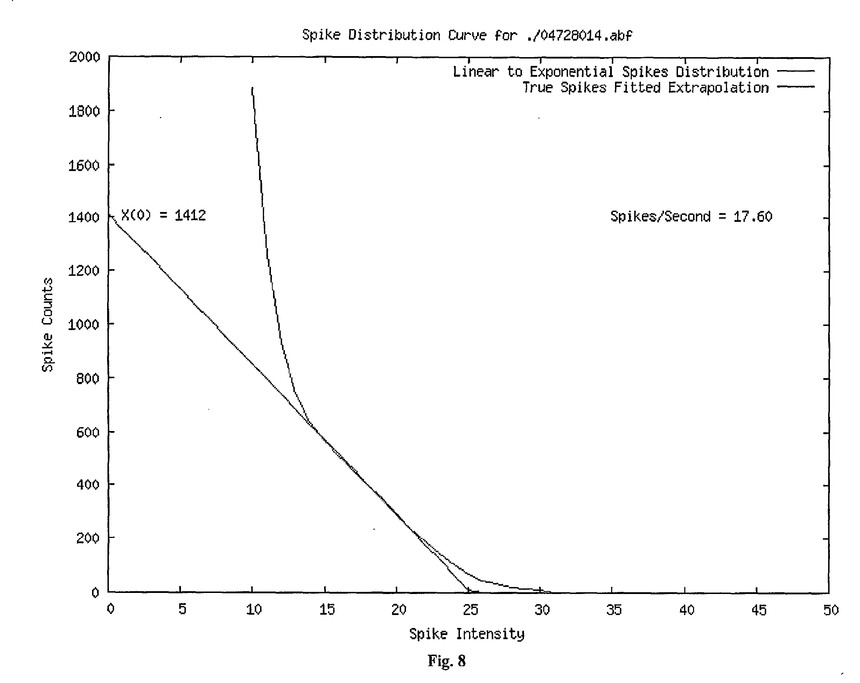

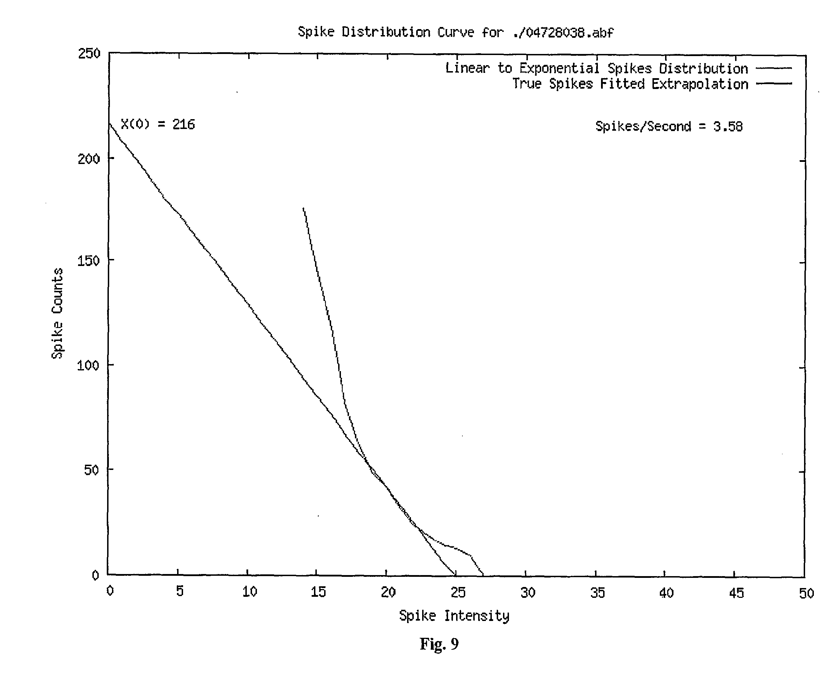

**Figure 8****.** The time-domain FSA shown in **Fig. 7** is used to extract fast time-domain features, such as "spike" blockade events. Automatically generated "spike" profiles are created in this process. One such plot is shown here for a radiated 9 base-pair hairpin, with a fraying rate indicated by the spike events per second (from the lower level sub-blockade). Results: the radiated molecule has more "spikes" which are associated with more frequent "fraying" of the hairpin terminus - the radiated molecules were observed with 17.6 spike events per second resident in the lower sub-level blockade, while for non-radiated there were only 3.58 such events (shown in **Fig. 9**).
**Figure 9****.** Automatically generated "spike" profile for the non-radiated 9 base-pair hairpin. Results: the non-radiated molecule had a much lower fraying rate, judging from its much less frequent lower-level spike density (3.58 such events per LLsec).

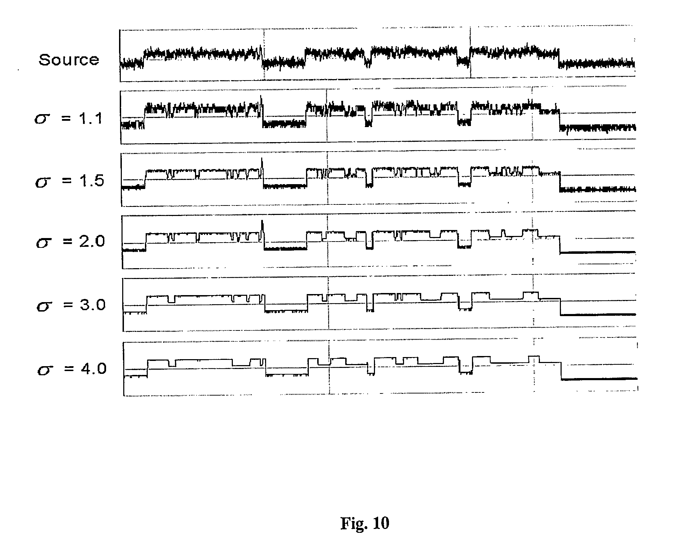

**Figure 10****.** This figure shows the blockade sub-level noise reduction capabilities of an HMM/EM x5 filter with gaussian parameterized emission probabilities. The sigma values indicated are multiplicative (i.e. the 1.1 case has standard deviation boosted to 1.1 times the original standard deviation). Sigma values greater than one blur the gaussians for the emission probabilities to greater and greater degree, as indicated for each resulting filtered signal trace in the figure. The levels are not preserved in this process, but their level transitions are highly preserved, now permitting level-lifetime information to be extracted easily via a simple FSA scan (that has minimal tuning, rather than the very hands-on tuning required for solutions purely in terms of FSAs).

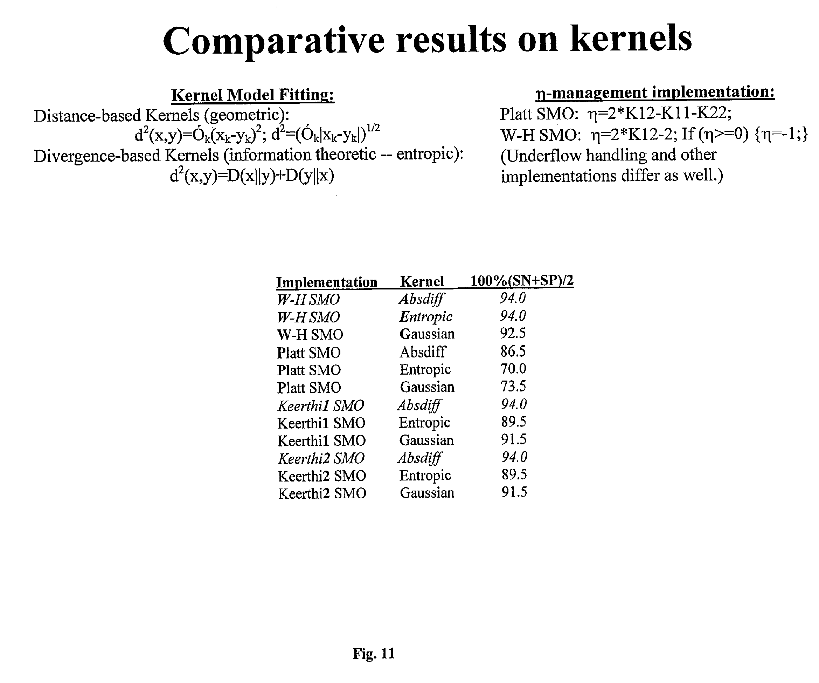

**Figure 11****.** This figure shows results from various SVM implementations. From the blockade data analyzed, it is typically found that SMO η-management done in the S-W way indicated, and divergence-based kernels, allow for the best, most stable, classifiers.

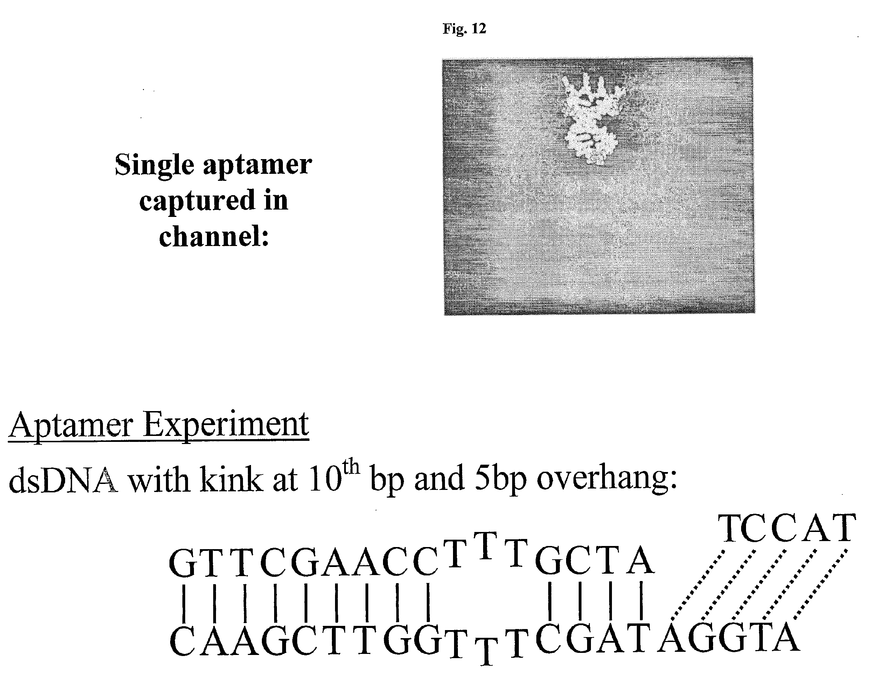

**Figure 12****.** The preliminary aptamer experiments performed are based on the dsDNA molecule shown here, with 5 base overhang used as a sensing moiety for the complimentary 5-base ssDNA molecule. The target 5-base ssDNA is introduced subsequent to obtaining a toggler-type capture of the aptamer molecule (properly annealed). The aptamer experiment is referred to above as a pseudo-aptamer experiment due to its simplification to a DNA annealing detection.

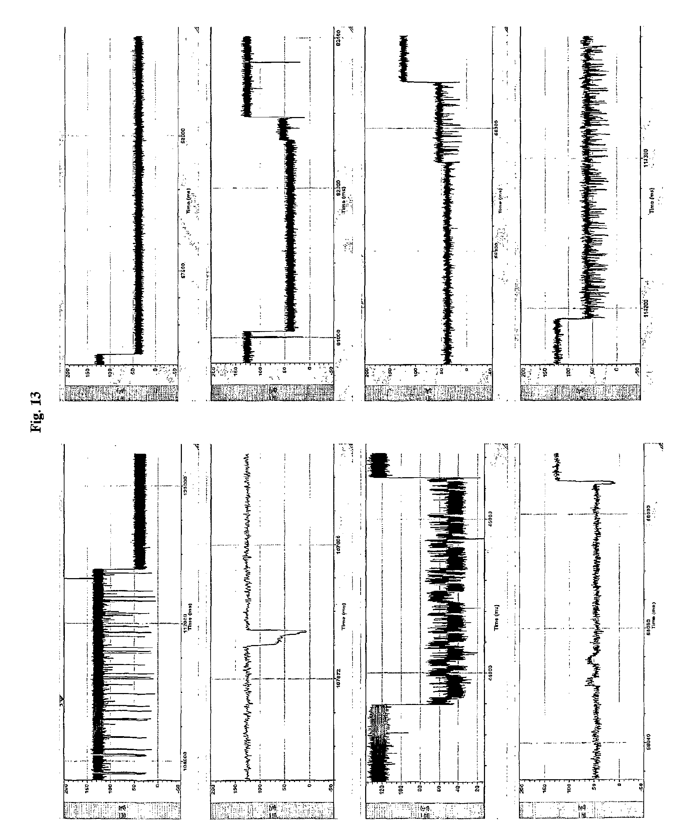

**Figure 13****.** Shows a collection of blockade signal traces. The short blockades in the upper left slide result from ssDNA translocation by the un-annealed ssDNA components. The long blockade in the upper left, and all right, panels is for capture with the overhang end entering first. The panel third down on the left shows the desired toggler-type signal. Each graph shows the level of current in picoamps over time in milliseconds.

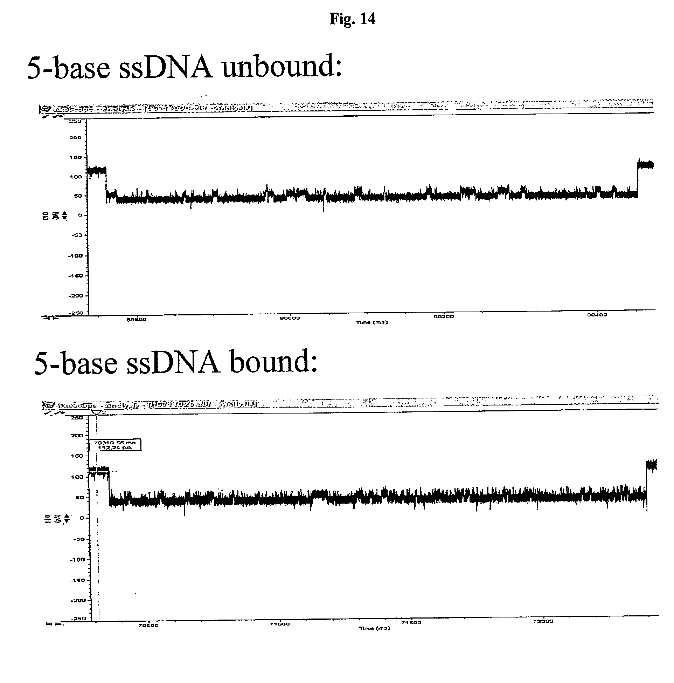

**Figure 14****.** Shows the modification to the toggler-type signal shortly after addition of 5-base ssDNA. The observed change is hypothesized to represent annealing by the complimentary 5-base ssDNA component, and thus detection of the 5-base ssDNA molecule. Each graph shows the level of current in picoamps over time in milliseconds.

### DETAILED DESCRIPTION OF EMBODIMENTS

The aspects of the present disclosure described here for biological channels are also applicable on solid-state platforms in a variety of incarnations - the underlying biophysics remains the same: a nanometer-scale ion channel together with ion-channel blockade analysis that uses a highly adaptable set of machine-learning-based signal analysis and pattern recognition methods. Going forward, solid-state channels will allow greater control of components of the device physics, so will play a prominent role. However, a complication with solid-state nanoscopic channels is that the technology for preparing them in solid-state media is still in its infancy. Preparing a single channel in a silicon nitride, for example, is fraught with difficulties in maintaining a stable channel -- partly because it is found that atomic flow occurs at the surface of the substrate that leads to closing of such small channels. Certain protein channels, on the other hand, are known to be very stable, and benefit from the fact that nature has designed them to self-assemble. Their biological origin has also led to their robust properties of heat dissipation, etc., upon polymer translocation, something that poses added complication in the solid-state setting. For these reasons, advances to date in nanoscopic-channel based detection have primarily involved single nanometer-scale protein channels ("nanopores") intercalated in lipid bilayer substrates. These biological channels provide a plethora of interesting research milestones with the information gained about the biological systems themselves.

A need continues to exist for improved understanding of specific and general membrane constituents that contribute to pore formation, as mentioned above, and for a new, nanopore-based cheminformatics characterization of the many currently known protein channels. Areas of research accessible to nanopore-based cheminformatics include: ion channel and pore-forming toxin characterization; assays of molecules that confer beneficial modification to channel function; and understanding the pore-forming mechanism. Agents that stimulate membrane damage include not only bacterial pore-forming toxins (PFTs), but some enveloped viruses, animal and plant toxins, and man-made molecules and environmental pollutants. PFTs are one of the most potent and versatile weapons wielded by invading microbes. A number of medically relevant pathogens are known to produce pore-forming proteins. In many cases, PFTs are significant virulence agents: markedly reduced virulence is observed in mutant bacterial strains that have lost their ability to produce their PFT. Antibodies against the alpha-hemolysin PFT, for example, have been shown to protect against various *S. aureus* infections, while immunization against the PFT of the human pathogen *Aeromonas hydrophilia* leads to protection. It should also be noted that, although PFTs directly have detrimental impact by destroying tissue cells and first-line immune cell defenses, their most deleterious impact is often less obvious, due to a wide spectrum of short- and long-range secondary reactions to the intracellular signaling confusion resulting from the lysed cells. It is hypothesized that the additional information obtained through practice of this disclosure will lead to a better understanding of these pore-forming constituents.

### Membrane Environment and Alternative Channels for use in Biosensing and Screening

The α-hemolysin channel of use in certain embodiments of the present invention is a protein heptamer, formed by seven identical 33 kD protein molecules secreted by *Staphylococcus aureus.* The total channel length is 10 nm and is comprised of a 5 nm *trans*-membrane domain and a 5 nm vestibule that protrudes into the aqueous *cis* compartment. The narrowest segment of the pore is a 1.5 nm-diameter aperture, see **Fig. 1a****.** By comparison, a single strand of DNA is about 1.3 nm in diameter. Given that water molecules are 0.15 nm in diameter, this means that one hydration layer separates ssDNA from the amino acids in the limiting aperture. This places the charged phosphodiester backbone, hydrogen bond donors and acceptors, and apolar rings of the DNA bases within one Debye length (3 Å in 1 M KCl) of the pore wall. Not surprisingly, DNA and RNA strongly interact with the α-hemolysin channel during translocation. Although dsDNA is too large to translocate, about ten base-pairs at one end can still be drawn into the large cis-side vestibule. This actually permits the most sensitive experiments to date, as the ends of "captured" dsDNA molecules can be observed for as long as desired to resolve features. For ssDNA translocation under normal operating conditions, approximately one nucleotide passes the limiting aperture of the channel every microsecond, and a vigorous effort is underway to find ways to slow down and control this translocation process.

Thus, in some aspects there is described herewith a nanopore detector, using either (i) a membrane protein that forms an aqueous transmembrane pore, or (ii) a solid state nanopore formed in a suitable solid state substrate. The alpha-hemolysin nanopore detector provides an explicit description of one of these aspects of the channel detection methods disclosed (see Fig. 1a). Alpha-hemolysin is a membrane protein that forms an aqueous transmembrane pore (similar aspects are possible with solid-state variants). In order for such a pore to form in a lipid bilayer it is necessary for lipid molecules to be laterally displaced. For microbial pore-forming toxins, the energy used to drive the pore-formation process is thought to be solely provided by conformational changes in the toxin molecules themselves (i.e., the process is ATP-independent). For alpha-hemolysin, the energy needed for the pore-formation is shown to be due to the oligomerization of toxin monomers to form the channel heptamer complex. Although pore-forming toxins, and membrane-permeabilizing molecules in general, have incredibly diverse sequence and structure, they all share in the same mechanism. They either directly intercalate into target membranes, or bind to particular target molecules in the membranes, and do so from a solution soluble monomeric form. They then assemble into multimeric, membrane-spanning pores. Attributes of the membrane, other than specific binding molecules, are often critical to this process, such as cholesterol-rich microdomains or lipid rafts. The role of cholesterol as a specific binding agent for individual formation events is well documented, and is required for pore-formation by many toxins (in natural setting, otherwise, channel formation can be activated by introduction of solvent, e.g., n-decane), including the Anthrax pore-forming toxin. But cholesterol-rich microdomains also play a role in channel formation in a non-specific manner. This is due to the microdomains acting as concentration platforms that can aid in the assembly of proto-channel multimers as is described for the aerolysin heptamer in Abrami, L., M. Fivaz, F.G. van der Goot, Surface dynamics of aerolysin on the plasma membrane of living cells, Int. J. Med. Microbiol. 290, 363-367 (2000). Once a proto-channel multimer has formed, such microdomains can also aid in the last step of transmembrane channel formation. This is due to the junctions between cholesterol-sphingolipid-rich domains and fluid-phase phosphoglyceride domains having locally favorable (weakened) bilayer characteristics that favor membrane penetration. The opposite effect is also known to be medically relevant: unknown membrane constituents, or the lack thereof, can block an alpha-hemolysin heptamer complex from inserting a transmembrane functional domain. This is found to be the case for human granulocytes, where the agent preventing channel formation is unknown.

Cholesterol not only factors into protein-channel based detection as a specific and general pore-formation co-agent, but also, paradoxically, as a membrane strengthening agent in that it allows for greater vibrational shock resistance in the bilayer. For this reason, even though cholesterol is not required for alpha-hemolysin channel formation in bilayers, it is still important in nanopore experiments purely as a bilayer-strengthening agent, which also serves to reduce the membrane noise contribution in the nanopore detector by approximately 35% (see Methods part of the EXPERIMENTAL Section).

### A New Method for Single Molecule Detection and Characterization

Angstrom precision structures for numerous DNA, RNA, and protein molecules have been revealed by X-ray diffraction analysis and NMR spectroscopy. These approaches rely upon average properties of very large numbers of molecules and are often biased towards crystallization and NMR conformer structures different from those present in solution under physiological conditions. With the introduction of single molecule analytical techniques in the early 1990's, however, new explorations into polymer structure and dynamics have begun. Others have shown that atomic force microscopy and laser tweezers, in particular, have permitted three direct measures of the force at the single molecule level: (1) the force required to break A•T or G•C base pairs, (2) the force required to extend single or double stranded DNA through distinct structural conformations, e.g., B form to S form DNA, etc., and (3) the forces exerted by polymerases working on polynucleotides.

Channel current based nanopore cheminformatics provides a new, incredibly powerful, method for biophysical and biochemical analysis. Single biomolecules, and the ends of biopolymers such as DNA, can now be examined in solution with nanometer-scale precision. In early studies, it was found that complete base-pair dissociations of dsDNA to ssDNA, "melting", could be observed for sufficiently short DNA hairpins. In later work (e.g., Winters-Hilt et al., 2003), the nanopore detector attained Angstrom resolution and was used to "read" the ends of dsDNA molecules, and was operated as a chemical assayer. In recent work, the nanopore detector is being used to observe the conformational kinetics at the termini of single DNA molecules. As part of the unique work in connection with the present disclosure, the nanopore is used to observe individual molecular interaction on-off binding events (see Results part of EXPERIMENTAL Section).

### Detection of short term binding and stationary phase.

There are important distinctions in how a nanopore detector can function: direct vs. indirect measurement and static or stationary vs. dynamic (possibly modulated) or non-stationary measurement.

A nanopore-based detector can *directly* measure molecular characteristics in terms of the blockade properties of individual molecules - this is possible due to the kinetic information that is embedded in the blockade measurements, where the adsorption-desorption history of the molecule (to the surrounding channel), and the configurational changes in the molecule itself, imprint on the ionic flow through the channel. This offers prospects for DNA sequencing and single nucleotide polymorphism (SNP) analysis. Nanopore methods may even have potential for *single-molecule* sequencing at some point in the future. Sequencing and SNP analysis, however, represent a small fraction of the immense potential of such a device. This is because it has now been discovered that a nanopore-based detector can also measure molecular characteristics *indirectly,* reporting on important binding kinetics in particular.

The nanopore-based detector works *indirectly* if it uses a reporter molecule that binds to certain molecules, with subsequent distinctive blockade by the bound-molecule complex. One example of this, with the established DNA experimental protocols, would be exploration of transcription factor binding sites via the different dsDNA blockade signals that occur with and without DNA-binding of a hypothesized transcription factor. Similarly, a channel-captured dsDNA "gauge" that is already bound to an antibody could provide a similar blockade shift upon antigen binding to its exposed antibody. The latter description provides the general mechanism for directly observing the *single molecule* antigen-binding affinities of any antibody. An unexpected result has been obtained, however, that simplifies this picture. It has been observed that antibodies can be directly captured and can produce a toggler-type signal on the nanopore detector, without the need for a linkage to a dsDNA gauge that is designed for that purpose. The hypothesized shift in blockade pattern (non-stationary) with antigen binding is also shown to occur in those preliminary studies (see Results part of EXPERIMENTAL Section).

The nanopore signal with the most utility and inherent information content is not merely the channel current signal for some static flow scenario, but one where that flow is modulated, at least in part, by the blockade molecule itself (with dynamic or non-stationary information, such as changing kinetic information). The modulated ion flow due to molecular motion and transient fixed positions (bound states) is much more sensitive to environmental changes than a blockade molecule (or open channel flow) where the flow is at some fixed blockade value (the rate of toggle between blockade levels could change, for example, rather than an almost imperceptible shift in a blockade signal residing at a single blockade value).

Part of the present novel device and method utility is not be based so much on the ionic flow (whether static or stationary), as changes in the modulations of that ionic flow. A transduction of the molecular binding and conformational kinetics to observed current measurement, for example, can be 100 times, or more, sensitive to alterations of that molecules environment (and thus its kinetics) than blockade observations based on molecules that blockade the channel at one fixed position.

In order to use nanoscopic pores to detect and identify single strands of DNA it is necessary to have automated signal processing. This is due to both the millisecond timescale desired for the classifications, and due to the immense number of signals to be analyzed. Even in careful studies of individual encoded molecules, where the channel blockade signals are obtained with seconds of measurement time, the presence of noise requires a typical data set to range from several hundred to several hundred thousand signals. With use of automated signal processing, the data size and processing speed no longer pose a fundamental problem, but they do pose fundamental constraints on the methods and processing architecture that can be employed.

**Fig. 1b** shows the prototype signal processing architecture developed in (Winters-Hilt et al., 2003). The processing is designed to rapidly extract useful information from noisy blockade signals using feature extraction protocols, wavelet analysis, Hidden Markov Models (HMMs) and Support Vector Machines (SVMs). For blockade signal acquisition and simple, time-domain, feature-extraction, a Finite State Automaton (FSA) approach is used that is based on tuning a variety of threshold parameters. The utility of a time-domain approach at the front-end of the signal analysis is that it permits precision control of the acquisition as well as extraction of fast time-scale signal characteristics. A wavelet-domain FSA (wFSA) is then employed on some of the acquired blockade data, in an off-line setting. The wFSA serves to establish an optimal set of states for on-line HMM processing, and to establish any additional low-pass filtering that may be of benefit to speeding up the HMM processing. HMMs can characterize current blockades by identifying a sequence of sub-blockades as a sequence of state emissions. See, e.g., Chung, S.H., J.B. Moore, L. Xia, L. S. Premkumar, and P. W. Gage, 1990, Characterization of single channel currents using digital signal processing techniques based on Hidden Markov models. Phil. Trans. R. Soc. Lond. B 329. 265-285; Chung, S-H., and P. W. Gage, 1998, Signal processing techniques for channel current analysis based on hidden Markov models, in Methods in Enzymology; Ion channels, Part B. P. M. Conn editior. Academic Press, Inc., San Diego, 420-437; Colquhoun, D., and F. J. Sigworth, 1995, Fitting and statistical analysis of single-channel products, in Single-channel recording, B. Sakmann and E. Neher editors, Second edition, Plenum Publishing Corp., New York, 483-587. The parameters of an HMM can then be estimated using a method called Expectation/Maximization. Although HMMs can be used to discriminate among several classes of input, multi-class computational scalability tends to favor their use as feature extractors (see Experimental section for further details). HMMs are well suited to extraction of aperiodic information embedded in stochastic sequential data such as in channel current blockades (or genomic sequences). Classification of feature vectors obtained by the HMM (for each individual blockade event) is then done using SVMs, an approach which automatically provides a confidence measure on each classification. SVMs are fast, easily trained, discriminators, for which strong discrimination is possible without the over-fitting complications common to neural net discriminators. SVMs strongly draw upon variational methods in their construction and are designed to yield the best estimate of the optimal separating hyperplane (for classifier, see **Fig. 2**) with confidence parameter information included (via hyperplane with margin optimization used in structural risk minimization). The SVM approach also encapsulates a significant amount of discriminatory information in the choice of kernel in the SVM, and a number of novel kernels have been developed (see the Experimental section for further details). In previous work (Winters-Hilt et al., 2003) novel, information-theoretic, kernels were successfully employed for notably better performance over standard kernels.

Different tools are employed at each stage of the signal analysis (as shown in **Fig. 1b**) in order to realize the most robust (and noise resistant) tools for knowledge discovery, information extraction, and classification. Statistical methods for signal rejection using SVMs are also be employed in order to reject extremely noisy signals (**Fig. 3**). Since the automated signal processing is based on a variety of machine-learning methods, it is highly adaptable to any type of channel blockade signal. This enables a new type of informatics (cheminformatics) based on channel current measurements, regardless of whether those measurements derive from biologically based or a semiconductor based channels.

### Software refinements are partly guided by the bioengineering device optimization.

The technical difficulty is to find molecules whose blockades interact with the channel environment, via short time-scale binding to the channel, or via inherent conformational changes in its high force environment, and that do so at timescales observable given the bandwidth limitations of the device. The 9 base-pair DNA hairpins previously studied have this property, so they can form the basis of a critical enhancement to the device, with one end performing the critical modulated blockade of the channel, and the other end possibly bound to any sensing moiety, such as an antibody. In the present disclosure, the sensing moieties are bound to something that is producing a very sensitive, rapidly changing, blockade signal due to its interaction kinetics with the channel environment (the DNA hairpin gauges, for example).

One of the features of the present disclosure is the indication that binding of antibody to the DNA hairpin gauges as the sensing moiety appears to be unnecessary, at least for some antibodies, as the antibodies themselves can enter the channel and provide the sensitive "toggling blockade" signal needed (see **Fig. 4**). We then see that binding of antigen can be observed as a change in that "toggling," as hypothesized (see **Fig. 5**).

A practical limitation of the nanopore device is that molecules to be detected, or specially designed blockade gauges (for indirect attachment) can blockade the channel and get "stuck" in one blockade state. This occurs for a 20 base-pair DNA hairpin that has the same 9 base-pair terminus as the "sensitive" DNA gauge described in the following: Winters-Hilt, S., "Nanopore detection using channel current cheminformatics," SPIE Second International Symposium on Fluctuations and Noise, 25-28 May, 2004; Winters-Hilt, S. and M. Akeson, "Nanopore cheminformatics," DNA and Cell Biology, Oct. 2004; Winters-Hilt et al., 2003; Winters-Hilt, S. 2003, Highly Accurate Real-Time Classification of Channel-Captured DNA Termini, Third International Conference on Unsolved Problems of Noise and Fluctuations in Physics, Biology, and High Technology, pg 355-368; and Vercoutere, W., S. Winters-Hilt, V. S. DeGuzman, D. Deamer, S. Ridino, J. T. Rogers, H. E. Olsen, A. Marziali, and M. Akeson, 2003, Discrimination Among Individual Watson-Crick Base-Pairs at the Termini of Single DNA Hairpin Molecules, Nucl. Acids Res. 31, pg 1311-1318). It no longer modulates the channel flow, being stuck in just one blockade state. But it has been discovered that the molecule can be re-excited to its telegraph signaling via a bead or some other form of attachment which is periodically tugged by a laser beam (or magnet). In this way, the larger DNA hairpin has it's blockade signal "re-awakened" to reveal binding kinetic information at its channel-captured end. Modulations to the channel environment appear to push an analyte into a highly sensitive interaction (and detection) regime vis-a-vis the channel detector (see **Fig. 6**).

### Using CCC Signal Analysis to advance understanding of the mechanisms of drug interaction.

The channel current cheminformatics (CCC) software needed for the present channel-based detection methodology furthers many objectives insofar as channel-based research is concerned, including high throughput screening assays for ion-channel modulatory agents. The drugs sought in high-throughput screening assays are meant to modify channel function in the case of ion channels with deleterious mutations, modify or disrupt channel function in the case of PFTs, or prevent channel-formation by PFTs in the first-place. There can be many subtleties to this search process. It is known that the antiviral drug amantadine, for example, inhibits the M2 pore function of type A influenza. The drug has a slow onset of action, however, which some hypothesize indicates its action may be due to allosteric influences on the channel. Such subtleties of drug action are another area where significant advances in understanding can be made via single channel experiments coupled with the CCC tools.

### Antibody Detection Interfaces for the Nanopore Detector.

In what follows, the properties of antibodies are given in detail to convey the subtle issues nanopore detection may help to resolve, and thereby provide improved opportunities for drug discovery. Other proteins, or biomolecules in general, will rarely have the same complexity as an antibody (or antibody-antigen interaction). Thus, in many respects, the nanopore detector analysis of antibody and antibody-antigen blockade observations are probing some of the most complex of protein-protein interactions.

The basic domain structure of antibody is an elongated globular domain consisting of beta-pleated sheets. Antigen binding occurs in the amino-terminal domain, while the effector functions are localized to the C-terminal domains. A flexible hinge joins the amino-terminal domains of the molecule (Fab consisting of two domains of the heavy chain and the entire light chain) with the carboxy-terminal portion (Fc). In the Experimental section we demonstrate that antibodies can be captured by the nanopore, most likely at their amino or carboxy terminus. Moreover, perturbations of the current blockade initiated by the capture are observed upon introduction of antigen. Presumably these are the result of antibody binding to the antigen. This interaction could affect the current blockade in two ways: 1. the antibody molecule may alter its conformation, or 2. the mass added by the (probably asymmetrical) addition of the antigen molecule, and consequent hinge flexibility, may affect the binding kinetics of the antibody in the pore. Both probably occur. The following describes the conformational perturbations in the antibody that occur on binding antigen, and how this may play a role in the initiation of antibody effector functions.

Immunoglobulin (Ig) is a bifunctional molecule. Antigen is bound by the Fab portion. Upon binding to antigen, a series of events are initiated by the interaction of the antibody Fc region with serum proteins and cellular receptors. Biological effects resulting from Fc interactions include activation of the complement cascade, binding of immune complexes by Fc receptors on various cells, and the induction of inflammation. Although effector molecules may bind to Fc of free antibody, binding is greatly enhanced if the antibody has bound antigen. Two different models have been proposed to explain this: associative and allosteric. The associative model explains enhanced binding as a result of multivalent interactions between the effector molecules and multiple Fc regions brought into close proximity by multiple antibodies binding to an antigen. The allosteric model proposes that there is a conformational change induced when antibody binds to antigen that makes the sites on Ig more accessible to the effector molecules. These models are not mutually exclusive and both mechanisms may play a role. There is strong evidence showing conformational changes within the Fab when antibody binds to antigen (reviewed below). Although it has not been shown that this conformational change is transmitted to the Fc region, flexibility of the hinge and the CH₂-CH₃ domain interface has clearly been demonstrated and associated with effector function.

The Ig molecule has structural flexibility that allows accommodation to the requirements of antigen binding and effector function. Changes in the structure of the Fab have been demonstrated on antigen binding. Due to difficulties in performing structural analyses on intact Ig molecules, it has only recently been possible to show whether conformational changes induced by antigen binding in IgG are transmitted to the Fc and effector molecule binding sites.

There is clear evidence of a conformational change on antigen binding by pentameric IgM. In the absence of antigen, IgM exists as a multimer yet binds to C1q with an affinity of only 10⁴ to 10⁵ M⁻¹. Complexed with antigen, the affinity increases to greater than 10⁷ M⁻¹, and a single IgM complexed to antigen can activate complement. Electron microscopy has been performed on IgM, both free and bound to antigen. In its uncomplexed form, the pentameric IgM assumes a star-shaped, planar conformation. When bound to antigen, the molecule folds, with the Fab regions dislocated out of the plane of the formed by the Fc's. This change in conformation either results in the exposure of additional complement-binding sites (associative model) or a change in the site itself that is more favorable to complement binding (allosteric model). In this case, the allosteric model is best supported by the data. Pentameric Fc isolated by the proteolytic removal of Fab binds C1q with the same affinity as uncomplexed intact IgM. Thus the effect of antigen binding cannot simply be the exposure of additional Clq-binding sites by movement of Fab arms, since the physical removal of these arms does not increase affinity.

There is structural flexibility inherent in the domain structure of the Ig molecule. Defined motions include: 1. Fc wagging, 2. Fab arm rotation, relative to the Fc, 3. Fab arm waving, alters the angle formed between the two Fab's relative to the Fc (T-shape vs Y-shape), and 4. Fab elbow bend, changing the angle between the Fv and the second domain of Fab (formed by CH1 and CL). All of these motions occur between different domains of the antibody; none involve changes in conformation within a domain. The greatest flexibility, and the major difference between Ig isotypes, is in the hinge region. Although it was originally thought, primarily on the basis of hinge-deletion mutants, that the structure of the hinge region was a primary influence on antibody effector function, more recent data suggests that hinge may have less of an effect on Fc-mediated functions than was initially assumed.

Interdomain flexibility of antibody probably serves several functions. Fab motions allow for bivalent antigen binding when individual epitopes are separated by variable distances and may even be necessary for optimal binding of monovalent antigen. Fc wagging probably increases accessibility of complement and Fc receptor binding motifs to their receptors, accomplished by minimizing steric hinderance that may occur on antigen binding. Finally, it is possible that interdomain flexibility transmits a conformational signal on antigen binding from the Fab to the Fc.

High resolution X-ray crystallographic studies have been performed with Fab and Fv fragments in the presence and absence of antigen. These studies have shown that the antibody combining site changes conformation to accommodate the antigen and bring hypervariable residues into contact with antigenic structures. For some antibodies these changes were minor, but in others major conformational changes were observed which not only affected the Fv, but were transmitted into the constant domains. Induced fit was also demonstrated in the structure of the antigens.
(i) The Fab fragment of the monoclonal anti-sweetener antibody NC6.8 was studied by others at 1.4 angstrom resolution. On complexation with antigen there were local changes affecting the positioning of both heavy and light chain CDRs. One of the consequences of this was to disrupt a hydrogen bond between Tyr H96 and Ser H98, causing CDR3 to expand and move 4.5 angstroms and a new hydrogen bond to form between Tyr H96 and the hapten. This caused V_{H} to flex and shorten. There was a concomitant lengthening of V_{L} by 10 angstroms, resulting in a decrease of 31° in the elbow bend between Fv and the first constant domain. The first constant domain was shifted as a unit so that the carboxyl terminus had moved 19 angstroms toward the Fv. The authors hypothesize that in an intact antibody, this allosteric change could be "relayed as tug (by tensile forces) on the segment connecting the Fab to the Fc region, perhaps altering the orientations of the constituents responsible for such effector functions as complement activation."
(ii) An antibody to the V3-loop of the HIV-envelope protein has been crystallized by others in the presence and absence of the synthetic peptide to which it binds. The presence of antigen induced a large rotation of V_{H} relative to V_{L} and influenced the orientation of the first constant domains as well.
(iii) X-ray crystal studies by others on intact immunoglobulins have been limited because of the difficulty in forming crystals caused by the inherent flexibility of the antibody molecule and because when crystals have formed the Fc units are disordered. Several intact antibodies have yielded a structure for the entire molecule including Fc. Interesting findings include: 1. an asymmetric configuration particularly within the CH₂ domain, resulting in somewhat independent conformations of the two CH₂ domains, and 2. flexibility of the CH₂ - CH₃ junction a site of many effector molecule interactions. On the basis of these 3-D studies it has been concluded by others that the structure illustrated the dynamic range inherent in the antibody and that the antibody is an assembly of units possessing a high degree of flexibility.
(iv) Electron microscopy and 2-D crystallography have demonstrated to others that both IgG and IgM are relatively planar structures in the absence of antigen, but that on antigen binding the Fab is dislocated from this plane. It has been proposed that this dislocation allows greater access of C1q and Fc receptors to the Fc portion of the antibody once it has bound antigen.

The flexibility of the antibody molecule is well established. There is also considerable data indicating that there are conformational changes induced by antigen binding.

### Aptamer Detection Interfaces for the Nanopore Detector.

Unlike the previous section on antibody biophysics, in this section details on the DNA (aptamer) biophysics are omitted for brevity.

An aptamer is a nucleic acid (DNA or RNA) that has strong binding affinity for a target protein, DNA/RNA, or other biomolecule. The binding affinity is selected for by PCR amplification of the most fit molecules, with fitness given by binding strength. Since the DNA hairpins with the desired toggle blockade properties can be viewed as "dumb aptamers," i.e., aptamers with no binding target, selection of DNA-hairpin variants *with the desired binding properties* is a logical starting point for the search process (constrained in both "toggler" captured-end parameters and *sensing moiety parameters*). Further detail is in the Results section of the EXPERIMENTAL Section below.

### A Novel Nanopore Device Implementation.

The relevant prior published literature has relied upon biosensing mechanisms that involve a nanometer-scale channel and blockades of that channel. Some of the literature has described modifications to the channel geometry and its binding properties (thus sensing properties) by introduction of auxiliary molecules. The auxiliary molecules that modify the channel sensing environment can be covalently bound (main case) or non-covalently bound to the channel (e.g., cyclodextrins non-covalently bound, or short ssDNA oligomers covalently bound). The design of these modifications is focused on providing alternative binding sites with no or scant attention paid to the role of the auxiliary molecules vis-a-vis their on-off binding with the channel itself. In the case of auxiliary molecules covalently bound to the channel, the sensitive on-off binding kinetics to the channel is eliminated entirely. In the case of the non-covalently bound (such as, e.g., the cyclodextrins), the information pertaining to the on-off binding of the auxiliary molecule is not pursued in its own right as the critically sensitive reporting mechanism that it entails. Instead, lengthy phases of fixed cyclodextrin conformation are sought. In essence, the channel blockade states associated with analyte detection, or non-detection, can all be described in the prior art as static blockades of the channel current at a fixed level. Typically, the binding or translocation of an analyte to the channel, or to the channel/auxiliary-molecule complex, is associated with a fixed reduction in the observed channel current.

Described herewith is a novel modification in the design and use of the auxiliary molecules. In particular, in the present disclosure, the auxiliary molecules produce a "toggling" blockade between several different levels (with two usually dominating). The resulting blockade signal for the auxiliary molecule by itself is no longer at approximately a fixed blockade level, but now consists of a telegraph-like blockade signal with stationary statistics. Upon binding of analyte to the auxiliary molecule the toggling signal is greatly altered, to one with different transition timing and different blockade residence levels. Building on this as a biosensing foundation requires sophisticated computational tools, such as Hidden Markov Models and Support Vector Machines, but offers at least a hundred-fold improvement to the sensitivity of the device. This is because the events of analyte bound versus non-bound (detected or not) can now be discerned with the much greater information in the multiple-level residencies and transition timings. This is in sharp contrast with the sparse information of non-bound having one blockade level and bound having another, single, blockade level. Given the noise in the system and the limited dynamic range for blockades of the open channel current, the device is greatly restricted if not endowed with the sensitive timing information. It has even been shown that minor environmental alterations to temperature, pH, etc., results in the toggle signal produced by "toggling" type auxiliary molecule being modified significantly -- in essence the channel with toggling-type auxiliary molecules can provide sensitive biosensing on the solution environment itself (which, in turn, is why the binding of target analyte to the toggling-type auxiliary molecule can be so sensitive).

Most of the prior art has been pursued by biochemists, and as such has focused on engineered protein pores (genetically, or via addition of auxiliary "carrier/adapter" molecules). The various incarnations of the engineered channels, and their binding (detection) properties, have almost entirely focused on events defined by a single (static) blockade level. What has not been appreciated, until now, is what can be accomplished with modern signal analysis and pattern recognition tools. In essence, by engineering a channel/auxiliary-molecule system with much greater complexity in the observed blockade signal, and then resolving that complexity with the aforementioned methods, a much better detector results. Now events are no longer defined by static blockade levels but by multiple blockade levels with internal transitions typically obeying stationary statistics. Also overlooked are various electronic modulation techniques for improving the effective bandwidth of the device.

Preliminary results clearly demonstrate higher sensitivity with toggling-type auxiliary molecules, as shown hereafter. This opens the door to a new, highly precise, means for examining the binding affinities between any two molecules, all while still in solution! In brief, the auxiliary molecule can be rigidly/covalently bound to one molecule of interest, and then exposed to a solution containing the other molecule of interest. The transitions between different stationary phases of blockade then correspond to the bound/unbound configuration between the two molecules of interest and reveals their binding kinetics (and binding strength). All the while, the toggling-type auxiliary molecule is producing its sensitive toggling blockade signal (even if modified with one species of analyte, or sensing moiety, rigidly/covalently bound to it).

Studies, described in what follows, were also done with toggling-type auxiliary molecules that also had sensing moieties at their uncaptured end (the end exposed to solution, not entering the channel). The molecules studied included antibodies and aptamers, and were chosen to demonstrate the specific utility of this device in drug candidate screening -- antibodies that bind strongly to target antigen are good antibodies, same for aptamers in many situations. Sometimes the drug is a toxin, so you want it to bind, but not for too long, etc. In the larger context of the channel device itself, the advanced computational tools offer the possibility of analyzing/characterizing the device channels themselves (typically pore-forming toxins, such as from staph or anthrax). The advanced computational tools also offer a platform for examining the efficacy of certain channel-forming toxins as cellular syringes for delivery of specific molecules and drugs to the cellular cytosol (such as antigen delivery to evoke a CTL response).

### EXPERIMENTAL

### Nanopore Detector Biophysics Methods

*"Nanopore experiments.* Each experiment is conducted using one α-hemolysin channel inserted into a diphytanoyl-phosphatidylcholine/hexadecane bilayer across a 25-micron-diameter horizontal Teflon aperture, as described previously in Akeson M, D. Branton, J.J. Kasianowicz, E. Brandin, D.W. Deamer, 1999, Microsecond Time-Scale Discrimination Among Polycytidylic Acid, Polyadenylic Acid, and Polyuridylic Acid as Homopolymers or as Segments Within Single RNA Molecules, Biophys. J. 77(6):3227-3233. Seventy microliter chambers on either side of the bilayer contains a conductive medium in the form of 1.0 M KCl buffered at pH 8.0 (10 mM HEPES/KOH) except in the case of buffer experiments where the salt concentration, pH, or identity may be varied. Voltage is applied across the bilayer between Ag-AgCl electrodes to provide a source for ionic flow. DNA control probes are added to the *cis* chamber at 10 or 20 µM final concentration. All experiments are maintained at room temperature (23 ± 0.1 °C), using a Peltier device.

*Controlling "Nanopore Noise Sources and Choice of Aperture.* The accessible detector bandwidth is delimited by noise resulting from 1/f (flicker) noise, Johnson noise, Shot noise, and membrane capacitance noise. In **Fig. 3a****,** upper right, the current spectral density is shown for the typical bilayer, an open α-hemolysin channel, and a channel with DNA hairpin blockade. For 1.0 M KCl at 23°C, the α-hemolysin channel conducts 120 pA under an applied potential of 120 mV. The thermal noise contribution at the 1 GΩ channel resistance has an RMS noise current of 0.4 pA, consistent with **Fig. 3a****.** Shot noise is the result of current flow based on discrete charge transport. During nanopore operation with 120pA current (with 10KHz bandwidth) there is, similarly, about 0.6 pA noise due to the discreteness of the charge flow. As with Johnson noise, the Shot noise spectrum is white, consistent with **Fig. 3a****.** The specific capacitance of lipid bilayers is approximately 0.8 µF/cm² (very large due to molecular dimensions), and the specific conductance is approximately 10⁻⁶ Ω⁻¹cm⁻². In order for bilayer conductance to produce less RMS noise current than fundamental noise sources (under the conditions above), the leakage current must be a fraction of a pA. This problem is solved by reducing to less than a 500µm² bilayer area, for which less than 0.6 pA leakage current results and for which total bilayer capacitance is at most 4pF. This indicates that a decrease in bilayer area by another magnitude is about as far as this type of noise reduction can go. Preliminary attempts to do this, however, lead to a very unpredictable toxin intercalation rate, among other difficulties. For the experiments considered here, the aperture ranges in size between 1 microns in diameter and 100 microns in diameter, where smaller apertures are used in the single channel experiments and larger apertures in the multi-channel experiments.

Nanopore-based detection is also limited by the kinetic nature (time-scale) of the blockade signal itself, since the molecular blockades typically involve binding and dissociation (analyte-channel binding, or antibody-antigen binding). It is hypothesized that it is possible to probe higher frequency realms than those directly accessible at the operational bandwidth of the channel current based device, or due to the time-scale of the particular analyte interaction kinetics, by modulated excitations. This can be accomplished by chemically linking the analyte or channel to an excitable object, such as a magnetic bead, under the influence of laser pulsations. In one configuration, the excitable object can be chemically linked to the analyte molecule to modulate its blockade current by modulating the molecule during its blockade. In another configuration, the excitable object is chemically linked to the channel, to provide a means to modulate the passage of ions through that channel. In a third experimental variant, the membrane is itself modulated (using sound) in order to effect modulation of the channel environment and the ionic current flowing though that channel. Studies involving the first, analyte modulated, configuration (**Fig. 6**), indicate that this approach can be successfully employed to keep the end of a long strand of duplex DNA from permanently residing in a single blockade state. Similar study of magnetic beads linked to antigen may be used in the nanopore/antibody experiments if similar single blockade level, "stuck," states occur with the captured antibody (at physiological conditions, for example). Likewise, this approach can be considered for increasing the antibody-antigen dissociation rate if it does occur on the time-scale of the experiment.

*Control probe design.* Since the five DNA hairpins studied in the prototype experiment have been carefully characterized, they are used in the antibody experiments as highly sensitive controls. The nine base-pair hairpin molecules examined in the prototype experiment share an eight base-pair hairpin core sequence, with addition of one of the four permutations of Watson-Crick base-pairs that may exist at the blunt end terminus, i.e., 5'-G•C-3', 5'-C•G-3', 5'-T•A-3', and 5'-A•T-3'. Denoted 9GC, 9CG, 9TA, and 9AT, respectively. (9GC has SEQ ID NO: 3; 9CG has SEQ ID NO: 4; 9TA has SEQ ID NO: 2; and 9AT has SEQ ID NO: 5.) The full sequence for the 9CG hairpin is 5' CTTCGAACGTTTTCGTTCGAAG 3' (SEQ ID NO: 4), where the base-pairing region is underlined. The eight base-pair DNA hairpin is identical to the core nine base-pair subsequence, except the terminal base-pair is 5'-G•C-3'. The prediction that each hairpin would adopt one base-paired structure was tested and confirmed using the DNA mfold server (http://bioinfo.math.tpi.edu/-mfold/dna/form1.cgi), which is based in part on data from SantaLucia J. 1998, A unified view of polymer, dumbbell, and oligonucleotide DNA nearest-neighbor thermodynamics, Proc. Natl. Acad. Sci. USA 95(4):1460-1465. A standardized aliquot of antibody is used as the control for antibody experiments once the kinetics of antibody capture and antigen-binding events are established and shown to be highly reproducible.

*Data acquisition.* Data is acquired and processed in two ways depending on the experimental objectives: (i) Using commercial software from Axon Instruments (Redwood City, CA) to acquire data, where current will typically be filtered at 50 kHz bandwidth using an analog low pass Bessel filter and recorded at 20 µs intervals using an Axopatch 200B amplifier (Axon Instruments, Foster City, CA) coupled to an Axon Digidata 1200 digitizer. Applied potential is 120 mV (*trans* side positive) unless otherwise noted. In some experiments, semi-automated analysis of transition level blockades, current, and duration are performed using Clampex (Axon Instruments, Foster City, CA). (ii) Using LabView-based experimental automation. In this case, ionic current is also acquired using an Axopatch 200B patch clamp amplifier (Axon Instruments, Foster City, CA), but it is then recorded using a NI-MIO-16E-4 National Instruments data acquisition card (National Instruments, Austin TX). In the LabView format, data is low-pass filtered by the amplifier unit at 50 kHz, and recorded at 20 µs intervals. In both fixed duty cycle (i.e., not feedback controlled) data acquisition approaches, the solution sampling protocol uses periodic reversal of the applied potential to accomplish the capture and ejection of single biomolecules. The biomolecules captured consist of antibodies and antigen, bound together or not, and in various orientations, and DNA control probes with stem-capture orientation and are added to the cis chamber typically in 20 µM concentrations. The time-domain finite state automaton used in the prototype is used to perform the generic signal identification/acquisition for the first 100 msec of blockade signal (Acquisition Stage, **Fig. 1b**). The effective duty cycle for acquiring 100 ms blockade measurements, when found to be sufficient for classification purposes, is adjusted to approximately one reading every 0.4 seconds by choice of analyte concentration. Further details on the voltage toggling protocol and the time-domain FSA are in **Fig. 7** and Winters-Hilt et al., 2003.

*Stability of the nanopore.* Because the nanopore is in a lipid bilayer, it is by definition a fluid structure and has a finite lifetime. Manipulations, especially adding antibody or antigen to the chamber, have the potential to disrupt the nanopore. If this occurs, it can greatly delay studies of the antibody-antigen interaction. But it should still be possible to perform these analyses. There is the potential for the replacement of lipid bilayers with more durable films, such as hybrid bilayer/solid-state systems where the bilayer rests on a thinly coated solid-state substrate. Alternatively, the protein-channel basis for the nanoscopic channel can be eliminated entirely, in favor of a purely solid-state channel (Li, J., D. Stein, C. McMullan, D. Branton, M. J. Aziz, and J. Golovchenko, Ion Beam Sculpting at nanometer length scales, Nature 412, 166-169 (2001)).

*Characterization under physiological buffer conditions.* The standard buffer condition for the nanopore detector is 1.0 M KCl with a pH of 8.0. This buffer was found to be most conducive to channel formation and to channels that do not gate. At significantly lower pH the channel is known to gate (Bashford, C.J., PORE-FORMING TOXINS: Attack and Defense at the cell surface, Cell. Biol. Mol. Lett. Vol. 6 No. 2A. 2001), if it even forms in the first place, which complicates use of the nanopore detector at physiological conditions (pH 7.0, 100mM NaCl). Since the pH of blood is usually in the range 7.35 to 7.45, and channel formation has been observed at 250 mM KCl, nanopore operation at the high pH and high salt end of the physiological range, relevant for antibody function in the bloodstream, may be possible with minimal alteration to the experimental parameters. Evaluation of antibody/antigen binding efficacy in a physiological buffer environment is particularly important if the nanopore/antibody detector is to be used for clinically relevant screening on the efficacy of antibodies to a given antigen.

If unable to alter buffer to physiological conditions, this will alter the on and off rates of antigen binding. But ELISA studies have shown that the antibodies still bind to the multivalent forms of the antigen in 1 M KCl, although binding is diminished, and it appears that one may be able to lower the KCl concentration to .25 M and still get pore formation. Thus while the validity of rate constants and other parameters of the interaction may be called into question, one may still be able to perform channel analyses, and other studies of antibody capture and alteration of channel blockade by antigen binding.

*Nanopore bio*-*sensor single-signal saturation.* Another limitation in the utility of the nanopore/antibody detector is that once antigen is bound by a channel-captured antibody it is very difficult to effect the release of that antigen. This is a complicating issue in acquiring a large sample of antibody-antigen binding observations. Attempts to "shake-off" the antigen by cycling to higher temperatures only serves to lead to added channel formation events - even after perfusion-elimination of solution toxin after the first channel formation. This is understood to result from higher mobility in the bilayer that allows resident toxin monomers to coalesce on the timescale of the experiment that would otherwise not do so. A buffer-based solution to this problem is already known from purifying antibodies through a column containing antigen, where the release of antibodies bound in the column is effected by perfusion with 1.0 M MgCl₂. This presents the possibility of weakening the antibody-antigen binding by similar choice of buffer to obtain large sample sets of binding events. The limitation of this is that the parameters will have likely deviated substantially from the physiological norm. Alternatively, a balanced stoichiometric ratio of antibody to antigen could be rapidly sampled, with lengthy sampling acquisitions only on antibody captures that occur without bound antibody and that then wait to observe antigen binding.

*Antibody*/*Antigen* -- *Design, Synthesis, and Purification.* For most of the experiments, a panel of native and genetically engineered antibodies to a well defined synthetic polypeptide antigen are used, (Y,E)-A-K. See Horgan, C., K. Brown, and S. H. Pincus. 1990. Alteration in heavy chain V region affects complement activation by chimeric antibodies. J. Immunol. 145:2527; Horgan, C., K. Brown, and S. H. Pincus. 1992a, Effect of H chain V region on complement activation by immobilized immune complexes, J. Immunol. 149:127; Horgan, C., K. Brown, and S. H. Pincus, 1992b, Variable region differences affect antibody binding to immobilized but not soluble antigen, Hum. Antibodies Hybridomas 3:153; and Horgan, C., K. Brown, and S. H. Pincus, 1993, Studies on antigen binding by intact and hinge-deleted chimeric antibodies, J. Immunol. 150:5400-5407. The antigen-binding characteristics, ability to form immune complexes, and effector functions of these antibodies have been carefully studied. Three different antibodies from this set are utilized in the experiments in this experimental effort. All have identical variable domains of murine origin, but one is a murine IgG1, one a human IgG1, and the other a human IgG4. These are designated T6, 10B, and B11, respectively. In addition to these antibodies, other antibodies are used to determine whether the ability of antibodies to be captured is a general phenomenon, and whether the blockade patterns obtained with antibodies are dependent on pI, isotype, species origin, or other predictable characteristics of the antibody or of the method of preparation.

All monoclonal antibodies are grown in tissue culture because ascites preparations are inflammatory exudates subjecting the antibodies to the potential of proteolytic digestion, attachment of complement components and so forth. Cells are either grown in medium containing fetal calf serum adsorbed on protein G to remove remaining Ig, or in serum free hybridoma medium. To test the effect of preparation method, murine IgG1 antibody is either purified by ammonium sulfate precipitation, antigen-affinity purification or protein G chromatography. This antibody has already been shown to be susceptible to capture. All other antibodies are routinely purified on protein G and eluted with 0.5M glycine-HCl pH 2.5, immediately neutralized, and dialyzed into phosphate buffered saline (PBS). Once antibodies are purified, they are run on SDS-PAGE to confirm purity and run on IEF prepoured gels (Biorad) to determine pI. Antigen binding is confirmed by ELISA in PBS and in 1 M KCl (so long as that buffer is used).

In addition to T6, 10B, and B11, the following antibodies are tested: murine and human monoclonal antibodies representing all of the IgG isotypes, murine IgG1 antibodies having different pIs (determined experimentally), and polyclonal protein A purified antibodies from human, rabbit, goat and mouse. The murine and human antibodies are obtained from an extensive collection of such antibodies. We have cell lines secreting multiple representatives of each isotype. Although the pIs of most of the antibodies may not be known, a collection of anti-DNA antibodies that have been tested (because of the ability of catioinic antibodies to deposit in kidneys) and have a range of PIs is used.

For the antigen-binding studies, different versions of (Y,E)-A-K are prepared which vary in molecular weight and valency. Previous studies have demonstrated that the epitope to which the antibodies bind may be represented by the synthetic peptide EYYEYEEY (Horgan et al., 1990, supra; Horgan et al., 1992a, supra; Horgan et al., 1992b, supra; and Horgan et al., 1993, supra). Thus we have the following antigens: 1. The synthetic polypeptide (Y,E)-A--K, which is highly multivalent and with different preparations has a molecular of either 50,000, 125,000, or 300,000 daltons. 2. EYYEYEEY which is monovalent and has a molecular weight of 1183, and 3. Ovalbumin, molecular weight 43,000 which has been conjugated to EYYEYEEY at ratios of 1, 3, and 10 peptides per molecule of OVA. These different antibody preparations allow study of the effect of antigenic mass and valency of binding upon the observations.

*Antibody Characterization Experiments*. The experiments characterize the blockade induced by the capture of antibody within the nanopore. The effects of antibody preparation and structural characteristics of the antibody are evaluated. Similar, reproducible, signal traces are believed to be found for antibody molecule blockades as in the DNA hairpin experiments. The complication is that the antibody molecule is more complex than the DNA hairpin molecule, and has several possible capture orientations instead of one. The objective, initially, is to enumerate the possible capture orientations. Because the immunoglobulin domain fold is an elongated barrel, it is likely that capture events will occur at the narrow ends of the domain, rather than along the side of the beta-barrel, since we have shown that albumin, a more globular molecule, cannot be captured. Thus it is possible that we may capture binding of domains consisting of individual chains, portions of both chains, within the Fab and Fc, and possibly, the carbohydrate attached to the heavy chain (although this is usually oriented internally and not surface accessible). Only some of these conformations will be able to bind antigen,(as demonstrated in the preliminary results). Thus, the overall objective in this case is to fully quantify the various channel captures of antibody alone. This information, combined with antigen binding data regarding the relative antigen binding capabilities of antibody in each conformation, allow one to begin to determine what blockade pattern is associated with different capture orientations. (To do this will require acquisition of sufficient amounts of the nanopore/antibody data to allow the machine learning software to be adequately trained.)

The capture of each antibody preparation should be studied by multiple events. Control software may be designed that automatically detects the capture event, collects data for a defined time (100 ms to 1 s depending on experiment), ejects the antibody from the nanopore by reversing the current, and then sets up to capture another antibody molecule. Additional software may be designed to classify the blockade signals obtained. In this way, one is able to collect data from several hundred capture events for each antibody preparation, classify them on the basis of channel blockade produced, and perform statistical analyses defining the rate for each type, and determine whether consistent results are obtained on a day-to-day basis, and determining whether the antibody pI, isotype, preparation, or species origin has an effect on the frequency of the capture modes.

### Channel Current Cheminformatics (Signal Analysis & Pattern Recognition) Methods.

Extraction of kinetic information begins with identification of the main blockade levels for the various blockade classes (off-line). This information is then used to scan through already labeled (classified) blockade data, with projection of the blockade levels onto the levels previously identified (by the off-line stationarity analysis) for that class of molecule. A time-domain FSA performs the above scan (**Fig. 8**), and uses the information obtained to tabulate the lifetimes of the various blockade levels. Once the lifetimes of the various levels are obtained, information about a variety of kinetic properties is accessible. If the experiment is repeated over a range of temperatures, a full set of kinetic data is obtained (including "spike" feature density analysis, as shown in **Fig. 9**). This data may be used to calculate kₒₙ and k_{off} rates for binding events, as well as indirectly calculate forces by means of the van't Hoff Arrhenius equation.

*Signal Preprocessing Details.* Each 100 ms signal acquired by the time-domain FSA consists of a sequence of 5000 sub-blockade levels (with the 20 µs analog-to-digital sampling). Signal preprocessing is then used for adaptive low-pass filtering. For the data sets examined, the preprocessing is expected to permit compression on the sample sequence from 5000 to 625 samples (later HMM processing then only required construction of a dynamic programming table with 625 columns). The signal preprocessing makes use of an off-line wavelet stationarity analysis (Off-line Wavelet Stationarity Analysis, **Figure 1b**).

*HMMs and Supervised Feature Extraction Details.* With completion of preprocessing, an HMM is used to remove noise from the acquired signals, and to extract features from them (Feature Extraction Stage, **Fig. 1b**). The HMM is, initially, implemented with fifty states, corresponding to current blockades in 1% increments ranging from 20% residual current to 69% residual current. The HMM states, numbered 0 to 49, corresponded to the 50 different current blockade levels in the sequences that are processed. The state emission parameters of the HMM are initially set so that the state j, 0 <= j <= 49 corresponding to level L = j+20, can emit all possible levels, with the probability distribution over emitted levels set to a discretized Gaussian with mean L and unit variance. All transitions between states are possible, and initially are equally likely. Each blockade signature is de-noised by 5 rounds of Expectation-Maximization (EM) training on the parameters of the HMM. After the EM iterations, 150 parameters are extracted from the HMM. The 150 feature vector components are extracted from parameterized emission probabilities, a compressed representation of transition probabilities, and use of *a posteriori* information deriving from the Viterbi path solution (further details in Winters-Hilt et al., 2003). This information elucidates the blockade levels (states) characteristic of a given molecule, and the occupation probabilities for those levels (**Fig. 3a****,** lower right), but doesn't directly provide kinetic information. The resulting parameter vector, normalized such that vector components sum to unity, is used to represent the acquired signal during discrimination at the Support Vector Machine stages.

A combination HMM/EM-projection processing followed by time-domain FSA processing allows for efficient extraction of kinetic feature information (e.g., the level duration distribution). **Fig. 10** shows how HMM/EM-projection might be used to expedite this process. The objective of the HMM/EM processing is to reduce level fluctuations, while maintaining the position of the level transitions. The implementation uses HMM/EM parameterized with emission probabilities as gaussians, which, for HMM/EM-projection, is biased with variance *increased by approximately one standard deviations* (see results shown). This method is referred to as HMM/EM projection because, to first order, it does a good job of reducing sub-structure noise while still maintaining the sub-structure transition timing. The benefit of this over purely time-domain FSA approaches is that the tuning parameters to extract the kinetic information are now much fewer and less sensitive (self-tuning possible in some cases).

The classification approach adopted in (Winters-Hilt et al., 2003) is designed to scale well to multi-species classification (or a few species in a very noisy environment). The scaling is possible due to use of a decision tree architecture and an SVM approach that permits rejection on weak data. SVMs are usually implemented as binary classifiers, are in many ways superior to neural nets, and may be grouped in a decision tree to arrive at a multi-class discriminator. SVMs are much less susceptible to over-training than neural nets, allowing for a much more hands-off training process that is easily deployable and scalable. A multiclass implementation for an SVM is also possible -- where multiple hyperplanes are optimized simultaneously. A (single) multiclass SVM has a much more complicated implementation, however, is more susceptible to noise, and is much more difficult to train since larger "chunks" are needed to carry all the support vectors. Although the "monolithic" multiclass SVM approach is clearly not scalable, it may offer better performance when working with small numbers of classes. The monolithic multiclass SVM approach also avoids a combinatorial explosion in training/tuning options that are encountered when attempting to find an optimal decision tree architecture. It was revealed in (Winters-Hilt et al., 2003), however, that the SVM's rejection capability often leads to the optimal decision tree architecture reducing to a linear tree architecture, with strong signals skimmed off class by class. This would prevent the aforementioned combinatorial explosion if imposed on the search space, and that should be the first architecture attempted for the signal classifications.

SVMs use variational methods in their construction and encapsulate a significant amount of discriminatory information in their choice of kernel. In reference (Winters-Hilt et al., 2003) one of the present inventors used novel, information-theoretic, kernels for notably better performance than standard kernels. The kernels studied were not limited to those satisfying Mercer's conditions, although they might, since they can be described as metrics "regularized" by incorporation as positive arguments in a decaying exponential. The commonly used Gaussian kernel, which does satisfy Mercer's conditions, has such an exponential form, and was outperformed in all cases studied by the entropic and variation kernels used in the prototype (Fig. 11, Winters-Hilt et al., 2003). The original motivation for working with the entropic kernel was to obtain a faster, more noise-resistant, kernel for information obtained via an HMM feature extractor, instead of the theoretically attractive, but less noise resistant, choice of directly integrating the two via a Fisher Kernel. This led to a general formulation where feature extraction was designed to arrive at probability vectors (i.e., discrete probability distributions) on a predefined, and complete, space of possibilities. (The different blockade levels, and their frequencies, for example.) This turns out to be a very general formulation, wherein feature extraction makes use of signal decomposition into a complete set of separable states. A probability vector formulation also provides a straightforward hand-off to the SVM classifiers since all feature vectors have the same length with such an approach. What this means for the SVM is that geometric notions of distance are no longer the best measure for comparing feature vectors. For probability vectors (i.e., discrete distributions), the best measures of similarity are the various information-theoretic divergences: Kullback -Leibler, Renyi, etc. By symmetrizing over the arguments of those divergences a rich source of kernels is obtained that works well with the types of probabilistic data obtained.

The SVM discriminators are trained by solving their KKT relations using the Sequential Minimal Optimization (SMO) procedure (Platt, 1998). A chunking variant of SMO also is employed to manage the large training task at each SVM node. The multi-class SVM training generally involves thousands of blockade signatures for each signal class. The data cleaning needed on the training data is accomplished by an extra SVM training round.

*Re*-*establishing the α-hemolysin channel- on a day-to-day basis presents a mayor complication to the pattern recognition task.* SVM classification in such circumstances faces weaker training convergence and poorer signal calling. For the blockade signals to be considered, two stabilization approaches are employed. (i) a passive stabilization approach that optimizes the kernels for high rejection. And, as needed, (ii) active, computationally-based, stabilization methods that track control signature samples that are intermixed with the target analyte signal (see description of DNA hairpin controls in the Nanopore Experimental Design).

*Development of a Class Independent HMM to extract kinetics information from channel current data.* Two, critical, engineering tasks must be addressed in a practical implementation of this objective: (i) the software should require minimal tuning; and (ii) feature extraction must be accomplished in approximately the same 100 ms time span as the blockade acquisition. (The latter, approximate, restriction was successfully implemented for the 300ms voltage-toggle duty cycle used in the prototype.) The feature extraction tools used to extract kinetic information from the blockade signals will include finite-state automata (FSAs), wavelets, as well as Hidden Markov Models (HMMs). Extraction of kinetic information from the blockade signals at the millisecond timescale for objectives (i) and (ii) are addressed by use of HMMs for level identification, HMM-EMs for level projection, and time-domain FSAs for processing of the level-projected waveform.

*Development of Class Dependent HMM*/*EM and NN algorithms to extract transient-kinetic information*. If separate HMMs are used to model each species, the multi-HMM/EM processing can extract a much richer set of features, as well as directly provide information for blockade classifications. The multiple HMM/EM evaluations, however, on each unknown signal as it is observed, represent a critical non-scaling engineering trade-off. The single-HMM/EM approach adopted in Winters-Hilt et al., 2003, on the other hand, scales well to multiple species classification (or a few species in a very noisy environment) because a *single,* HMM/EM was used, and the entire discriminatory task was passed off to a decision tree of Support Vector Machines (SVMs). Another benefit of incorporating SVMs for discrimination was that they provided a robust method for rejecting weak data. The software design in some aspects of this disclosure retains the clear benefits of the SVM-based classification approach, but now incorporates a richer set of information from multi-HMM/EM processing. The challenge in doing so is to perform the extensive feature extraction in the allotted sampling timeframe. Where real-time processing can't be accomplished with species-specific multi-HMM/EM processing, an alternative approach is to augment the feature extraction with neural net (NN) *feature extraction* methods (not discrimination) that can learn and extract features on-line. In this approach, information about the level states, and their time-constants, is obtained in a much cruder form.

*Development of multi-class SVMs and develop SVM Kernels specialized for discrete probability feature vectors.* This signal processing classifies molecular blockades based on kinetic profiles, and recognizes *transitions* between kinetic profiles. Initially this is accomplished by extending the Support Vector Machine (SVM) Decision Tree approach. There are two, critical, engineering hurdles for a practical implementation of this aim: (i) automation in training of the SVM architecture; and (ii) optimization over a class of relevant SVM Kernels. For problem (i), a great deal of automation in training has already been accomplished in the construction of the prototype SVM Decision Tree. In that effort, the decision tree training and testing problem was reduced to specifying the topology of the tree and the location of the files used in training each of its nodes. Scripts were written to process a specified list of different decision trees and distribute the processing for those trees over a network of computers. If the SVM Decision Tree approach fails at the training stage, an alternate approach involving a monolithic multiclass SVM (with no decision tree) is possible. For problem (ii), the search for a suitable kernel, the proposed SVM classifier implementation significantly benefits from the extended class of divergence kernels pioneered in developing the prototype SVM. In that work the divergence kernels were designed to match with the probability vector data obtained with the feature extraction approach, and appear to be very well suited to signal analysis on channel current blockades.

### Antibody Experimental Results.

The first introduction of antibody into the nanopore detector analyte chamber was meant to examine the time-scale before disruption of the channel current. This was expected to occur because the antibody had been designed to target the epitopes of the alpha-hemolysin monomer. The unexpected result was that the antibody appeared to be drawn into the channel, just like dsDNA, with result a similar binding-kinetics "telegraph" signal in the channel current. This experiment has since been repeated hundreds of times, with a variety of different antibodies. Most of the antibodies examined have identical variable domains of murine origin.

Preliminary results indicate that single antibody molecules can be drawn into the nanopore detector in a manner analogous to the DNA hairpin studies. In those DNA hairpin blockade studies it was found that DNA hairpins, with stem lengths ranging between eight base-pairs and twelve base-pairs, produced blockade traces with transitions between a limited number of blockade states that were highly sensitive to changes in voltage and temperature. Analysis of those, reproducible, DNA hairpin signal traces led to a consistent picture for the binding and conformational kinetics for the captured ends of those molecules (Winters-Hilt et al., 2003; Vercoutere, W., S. Winters-Hilt, V. S. DeGuzman, D. Deamer, S. Ridino, J. T. Rogers, H. E. Olsen, A. Marziali, and M. Akeson, 2003, Discrimination Among Individual Watson-Crick Base-Pairs at the Termini of Single DNA Hairpin Molecules, Nucl. Acids Res. 31, pg 1311-1318; Winters-Hilt, S. 2003, Highly Accurate Real-Time Classification of Channel-Captured DNA Termini, Third International Conference on Unsolved Problems of Noise and Fluctuations in Physics, Biology, and High Technology, pg 355-368; Winters-Hilt, S. and M. Akeson, "Nanopore cheminformatics," DNA and Cell Biology, Oct. 2004; Winters-Hilt, S., "Nanopore detection using channel current cheminformatics," SPIE Second International Symposium on Fluctuations and Noise, 25-28 May, 2004). As with those DNA studies, where only a single channel was conducting current through a bilayer, an antibody blockade can be seen as a clearly discernible event (see **Fig. 4**). Instead of the open channel current being the baseline reference in the nanopore detector, however, there is now the possibility that the nanopore/antibody blockade may form a reference channel current signal in its own right. An antigen-binding event then perturbs the channel current signal, resulting from the antibody blocking the channel alone, and is the basis of the antibody-antigen binding-event observation (**Fig. 5**). The lifetimes of the different channel blockades associated with the antibody-antigen binding (and dissociation) can then be used to understand the kinetics of that binding, including its strength, with implications as to the efficacy of the antibody for that antigen.

The blockade signals for murine IgG1 (denoted T6) has been examined most extensively thus far. The antibody exhibits more than one blockade signal, suggesting that the different parts of the molecule can be drawn in. Since the immunoglobulin domain fold is an elongated barrel, it is possible that capture events could occur at the narrow ends of the domain, as individual chains, or portions of both chains, within the Fab and Fc, and possibly, the carbohydrate attached to the heavy chain. **Figure 4** shows other blockade signals that have been observed, along with images of possible capture orientations that might accompany them (all images are overlaid to scale).

It was hypothesized that the antibody blockade signal should alter shortly after introduction of antigen if antigen binding were to occur and alter the channel blockade. This hypothesis was partly based on previous results with DNA hairpin blockades, that also exhibited the telegraph blockade signal, and that were found to be very sensitive to environmental conditions. This hypothesis proves to be correct, as **Fig. 5** shows upon addition of moderately high concentration (100 µg/ml) of 200kDa multivalent synthetic polypeptide (Y,E)-A-K. The timescale before the blockade signals are altered is also interesting, it can range from seconds to minutes, which may help to resolve which blockade signal goes with what type of blockade in **Fig. 4****.**

Once antigen is bound by a channel-captured antibody it is very difficult to effect the release of that antigen. This is a complicating issue when seeking to obtain a large sample of antibody-antigen binding observations. Another complicating issue is that an antibody might form a blockade that does not exhibit the multi-level kinetic signal, residing in a "stuck" state instead. This is already a problem that is observed to occur for longer dsDNA molecules. The solution may be to periodically knock the system to keep it out of its "stuck" state in order to have it continue to exhibit its terminus binding kinetics. Recent results with a laser excitation of a magnetic bead that is attached to a DNA hairpin with 20 base-pair stem length (attachment at the hairpin loop) is shown in **Fig. 6****.** This preliminary result holds great promise for keeping analyte blockades in their most sensitive regime, as well as offering prospects for injecting modulatory side information.

Examples of excitable objects include microscopic beads, magnetic and nonmagnetic, and fluorescent dyes and the like. Bead attachments can couple in excitations passively from background thermal (Brownian) motions, or actively, in the case of magnetic beads, by laser pulsing and laser-tweezer manipulation. Dye attachments can couple excitations via laser or light (UV) excitations to the targeted dye molecule. Large, classical, objects, such as microscopic beads, provide a method to couple periodic modulations into the single-molecule system. The direct coupling of such modulations, at the channel itself, avoids the low Reynolds number limitations of the nanometer-scale flow environment. For rigid coupling on short biopolymers, the overall rigidity of the system also circumvents limitations due to the low Reynolds number flow environment. Similar consideration also come into play for the dye attachments, except now the excitable object is typically small, in the sense that it is usually the size of a single (dye) molecule attachment. Excitable objects such as dyes must contend with quantum statistical effects, so their application may require time averaging or ensemble averaging, where the ensemble case involves multiple channels that are observed simultaneously - which relates to the platform of the multi-channel configuration of the experiment. Modulation in the third, membrane-modulated, experiment also avoids quantum and low Reynolds number limitations. In all the experimental configurations, a multi-channel platform may be used to obtain rapid ensemble information. In all cases the modulatory injection of excitations may be in the form of a stochastic source (such as thermal background noise), a directed periodic source (laser pulsing, piezoelectric vibrational modulation, etc.), or a chirp (single laser pulse or sound impulse, etc.). If the modulatory injection coincides with a high frequency resonant state of the system, low frequency excitations may result, i.e., excitations that can be monitored in the usable bandwidth of the channel detector. Increasing the effective bandwidth of the nanopore device greatly enhances its utility in almost every application.

*Biotechnology Application:* Increasing the effective bandwidth of the nanopore device greatly enhances its utility in almost every application, particularly those, such as DNA sequencing, where the speed with which blockade classifications can be made (sequencing) is directly limited by bandwidth restrictions.

### Aptamer Experimental Results.

An aptamer is a nucleic acid (DNA or RNA) that has strong binding affinity for a target protein, DNA/RNA, or other biomolecule. The binding affinity is selected for by PCR amplification of the most fit molecules, with fitness given by binding strength. Since the DNA hairpins with the desired toggle blockade properties can be viewed as "dumb aptamers," i.e., aptamers with no binding target, selection of DNA-hairpin variants with the desired binding properties is a logical starting point for the search process (constrained in both "toggler" captured-end parameters and sensing moiety parameters). **Fig. 12** shows an aptamer with a "toggler"-type nine-base-pair stem captured end and a ssDNA-overhang for complement ssDNA detection (a "pseudo-aptamer"). **Fig. 13** shows a collection of blockade signal traces. The short blockades in the upper left slide of **Fig. 13** result from ssDNA translocation by the un-annealed ssDNA components. The long blockade in the upper left, and all right, panels is for capture with the overhang end entering first. The panel third down on the left shows the desired toggler-type signal. **Fig. 14** shows the modification to the toggler-type signal upon addition of 5-base ssDNA. The observed change is hypothesized to represent annealing by the complimentary 5-base ssDNA component, and thus detection of the 5-base ssDNA molecule.

### SEQUENCE LISTING FREE TEXT

SEQ ID NO: 1: DNA hairpin used in antibody experiments as a control.
SEQ ID NO: 1: DNA hairpin used in antibody experiments as a control.
SEQ ID NO: 2: DNA hairpin used in antibody experiments as a control.
SEQ ID NO: 3: DNA hairpin used in antibody experiments as a control.
SEQ ID NO: 4: DNA hairpin used in antibody experiments as a control.
SEQ ID NO: 5: DNA hairpin used in antibody experiments as a control.
SEQ ID NO: 6: Synthetic peptide to which the antibodies may be represented.
SEQ ID NO: 7: Peptide used for antigen binding studies.

## Claims

1. A method for molecular analysis comprising
receiving a blockade channel current signal **characterized by** continuous transitions between a plurality of levels and the transitions between levels having different transition timings, wherein the blockade channel current signal is carried by an ionic current flowing through a partially-blockaded nano-scale membrane channel defined by a membrane which partitions a conductive medium wherein the nano-scale membrane channel is partially blockaded by the presence of a single reporter molecule being an antibody non-covalently bonded to the membrane channel, wherein the blockade signal for the single reporter molecule is not at a fixed blockade level, wherein the blockade signal consists of a telegraph-like blockade signal with stationary statistics,
exposing the channel to a test condition during ionic current flow while extracting from the blockade channel current signal, a set of one or more pattern features to establish over a period of time either a blockade channel current signal pattern or a change in the blockade channel current signal pattern;
wherein the test condition is comprised of the introduction of an antigen to the conductive medium, and wherein the antigen specifically binds to the reporter molecule, such that upon specific binding of at least one antigen to the reporter molecule the toggling signal is greatly altered, to one with different transition timing and different blockade residence levels.

2. A method according to claim 1, further comprising
comparing the blockade channel current signal pattern, or the change in the blockade channel current signal pattern, to at least one blockade channel current signal pattern, or at least one change in the blockade channel current signal pattern, associated with at least one known condition, to thereby correlate the test condition with the known condition.

3. A method according to claim 1, further comprising
correlating the blockade channel current signal pattern or the change in the blockade channel current signal pattern with a characterization of interaction between the antigen and the reporter molecule.

## Patentansprüche

1. Verfahren zur Molekülanalyse, umfassend
Empfangen eines Blockadekanalstromsignals, charakterisiert durch kontinuierliche Übergänge zwischen einer Vielzahl von Niveaus, und wobei die Übergänge zwischen den Niveaus unterschiedliche Übergangszeiten aufweisen, wobei das Blockadekanalstromsignal durch einen Ionenstrom realisiert wird, der durch einen teilweise blockierten Membrankanal im Nano-Maßstab fließt, definiert durch eine Membran, die ein leitfähiges Medium unterteilt, wobei der Membrankanal im Nano-Maßstab teilweise durch die Gegenwart eines einzelnen Reportermoleküls blockiert wird, welches ein Antikörper ist, der nicht kovalent an den Membrankanal gebunden ist, wobei das Blockadesignal für das einzelne Reportermolekül nicht bei einem festen Blockadeniveau liegt, wobei das Blockadesignal aus einem telegrafenähnlichen Blockadesignal mit feststehenden Kenngrößen besteht,
Einwirkenlassen einer Testbedingung auf den Kanal während einem Ionenstromfluss, während von dem Blockadekanalstromsignal ein Satz von einem oder mehreren Mustermerkmalen gewonnen wird, um über einen Zeitraum entweder ein Blockadekanalstromsignalmuster oder eine Veränderung bei dem Blockadekanalstromsignalmuster zu ermitteln;
wobei die Testbedingung die Einbringung eines Antigens in das leitfähige Medium umfasst und wobei das Antigen spezifisch an das Reportermolekül bindet, so dass über spezifische Bindung von mindestens einem Antigen an das Reportermolekül das Schaltsignal stark zu einem mit unterschiedlicher Übergangszeit und unterschiedlichen Blockadeverweilniveaus verändert wird.

2. Verfahren gemäß Anspruch 1, ferner umfassend:
Vergleichen des Blockadekanalstromsignalmusters oder der Veränderung bei dem Blockadekanalstromsignalmuster mit mindestens einem Blockadekanalstromsignalmuster oder mindestens einer Veränderung bei dem Blockadekanalstromsignalmuster im Zusammenhang mit mindestens einer bekannten Bedingung, um dabei die Testbedingung mit der bekannten Bedingung zu korrelieren.

3. Verfahren gemäß Anspruch 1, ferner umfassend
Korrelieren des Blockadekanalstromsignalmusters oder der Veränderung bei dem Blockadekanalstromsignalmuster mit einem Charakteristikum einer Wechselwirkung zwischen dem Antigen und dem Reportermolekül.

## Revendications

1. Procédé d'analyse moléculaire comprenant :
la réception d'un signal de blocage du courant de canal, **caractérisé par** des transitions continues entre une série de niveaux et les transitions entre des niveaux ayant des rythmes différents, où le signal de blocage du courant de canal est réalisé par un courant ionique s'écoulant au travers d'un canal membranaire nanométrique partiellement bloqué défini par une membrane qui sépare un milieu conducteur dans lequel le canal membranaire nanométrique est partiellement bloqué par la présence d'une molécule reporter unique, qui est un anticorps lié de manière non covalente au canal membranaire, où le signal de blocage pour la molécule reporter unique n'est pas à un niveau fixé de blocage, où le signal de blocage consiste en un signal de blocage de type télégraphe avec statistiques stationnaires,
l'exposition du canal à des conditions de test pendant l'écoulement du courant ionique tout en extrayant du signal de blocage du courant de signal, un ensemble d'une ou de plusieurs caractéristiques de motif pour établir sur une certaine période, un motif de signal de blocage du courant de canal ou un changement du motif du signal de blocage du courant de canal ;
où les conditions de test comprennent l'introduction d'un antigène dans le milieu conducteur, et où l'antigène se lie spécifiquement à la molécule reporter de sorte que lors de la liaison spécifique d'au moins un antigène à la molécule reporter, le signal alternant est fortement altéré en un signal ayant un rythme différent de transition et des seuils différents de blocage.

2. Procédé selon la revendication 1, comprenant en outre :
la comparaison du motif de signal de blocage du courant de canal, ou du changement du motif de signal de blocage du courant de canal, avec au moins un motif de signal de blocage du courant de canal, ou au moins un changement de motif de signal de blocage du courant de canal, associé à au moins une condition connue, pour ainsi corréler la condition de test à la condition connue.

3. Procédé selon la revendication 1, comprenant en outre :
la corrélation du motif de signal de blocage du courant de canal ou du changement du motif de signal de blocage du courant de canal avec une caractérisation de l'interaction entre l'antigène et la molécule reporter.


### SEQUENCE LISTING

<110> Board of Supervisors of Louisiana State University and Agricultural and Mechanical college Acting for the university of New Orleans
   Board of Supervisors of Louisiana State University and Agricultural and Mechanical College Acting for the Louisiana State university Health Sciences Center in New Orleans
<120> CHANNEL CURRENT CHEMINFORMATICS AND BIOENGINEERING METHODS FOR IMMUNOLOGICAL SCREENING, SINGLE-MOLECULE ANALYSIS, AND SINGLE-MOLECULAR-INTERACTION ANALYSIS
<130> S-0872-A+-PCT
<150> 60/616,274, 60/616,275, 60/616,276 and 60/616,277
   <151> 2004-10-06
<160> 6
<170> PatentIn version 3.3
<210> 1
   <211> 20
   <212> DNA
   <213> Artificial
<220>
   <223> DNA hairpin used in antibody experiments as a control.
<400> 1
   gtcgaacgtt ttcgttcgac 20
<210> 2
   <211> 22
   <212> DNA
   <213> Artificial
<220>
   <223> DNA hairpin used in antibody experiments as a control.
<400> 2
   tttcgaacgt tttcgttcga aa 22
<210> 3
   <211> 22
   <212> DNA
   <213> Artificial
<220>
   <223> DNA hairpin used in antibody experiments as a control.
<400> 3
   gttcgaacgt tttcgttcga ac 22
<210> 4
   <211> 22
   <212> DNA
   <213> Artificial
<220>
   <223> DNA hairpin used in antibody experiments as a control.
<400> 4
   cttcgaacgt tttcgttcga ag 22
<210> 5
   <211> 22
   <212> DNA
   <213> Artificial
<220>
   <223> DNA hairpin used in antibody experiments as a control.
<400> 5
   attcgaacgt tttcgttcga at 22
<210> 6
   <211> 8
   <212> PRT
   <213> Artificial
<220>
   <223> Synthetic peptide to which the antibodies may be represented.
<400> 6
   Glu Tyr Tyr Glu Tyr Glu Glu Tyr
   1               5